(19) Europäisches Patentamt
European Patent Office
Office européen des brevets

(11) **EP 3 049 440 B1**

(12) **EUROPEAN PATENT SPECIFICATION**

(45) Date of publication and mention
of the grant of the patent:
**25.03.2020 Bulletin 2020/13**

(51) Int Cl.:
***C07K 16/28*** *(2006.01)* ***C07K 16/32*** *(2006.01)*

(21) Application number: **14782039.3**

(86) International application number:
**PCT/US2014/057432**

(22) Date of filing: **25.09.2014**

(87) International publication number:
**WO 2015/048272 (02.04.2015 Gazette 2015/13)**

(54) **V-C-FC-V-C ANTIBODY**

V-C-FC-V-C-ANTIKÖRPER

ANTICORPS V-C-FC-V-C

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.09.2013 US 201361882428 P**

(43) Date of publication of application:
**03.08.2016 Bulletin 2016/31**

(73) Proprietor: **Amgen Inc.**
**Thousand Oaks, California 91320-1799 (US)**

(72) Inventors:
• **MICHAELS, Mark, L.**
**Thousand Oaks, California 91320-1799 (US)**
• **BORGES, Luis, G.**
**Thousand Oaks, California 91320-1799 (US)**
• **YAN, Wei**
**Thousand Oaks, California 91320-1799 (US)**

(74) Representative: **Schiweck Weinzierl Koch**
**Patentanwälte Partnerschaft mbB**
**Ganghoferstraße 68 B**
**80339 München (DE)**

(56) References cited:
**WO-A1-2011/028952 WO-A1-2012/025525**

**Description**

**FIELD**

This application is in the field of antibody engineering.

**BACKGROUND**

Bispecific antibodies have promise as therapeutics in a variety of indications. Bispecific antibodies having a standard IgG format can be challenging to produce because they include four different polypeptide chains. The efficacy of a smaller, more easily-produced bispecific molecule has been clinically demonstrated in non-Hodgkin's lymphoma. Bargou et al. (2008), Science 321(5891): 974-977. Daily administration was used to achieve these results, presumably because of the short *in vivo* half life of this small, single chain molecule. *Id.* Immunoglobulin compositions that coengage antigens are known from WO 2011/028952 and activatable bispecific antibodies are described in WO 2012/025525. However, there remains a need in the art for bispecific therapeutics with favorable pharmacokinetic properties, as well as therapeutic efficacy and a format that makes them straightforward to produce.

**SUMMARY**

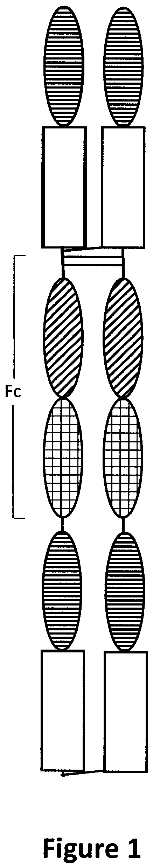

Herein is described an antibody comprising two polypeptide chains: the first polypeptide chain comprising the following regions, which may or may not be separated linkers, in the following order in an N- to C-terminal direction: V-1---C-1---hinge---CH2-1---CH3-1---V-2---C-2; and the second polypeptide chain comprising the following regions, which may or may not be separated by linkers, in the following order in an N- to C-terminal direction: V-3---C-3---hinge---CH2-2---CH3-2---V-4---C-4; wherein the V-1 to V-4 are immunoglobulin variable regions; wherein the C-1 to C-4 are immunoglobulin constant regions, and the C-1 and C-3 and also the C-2 and C-4 form pairs of a heavy chain constant region 1 (CH1) and a light chain constant region (CL) and/or if C-1 is a CH1, then C-3 is a CL and vice versa, and if C-2 is a CH1, then C-4 is a CL and vice versa; and wherein the CH2-1 and CH2-2 are each a heavy chain constant region 2 and the CH3-1 and CH3-2 are each a heavy chain constant region 3. *See* Figure 1. In one aspect, the V-1 can be a heavy chain variable region (VH), the C-1 can be a CH1, the V-2 can be a VH, the C-2 can be a CH1, the V-3 can be a light chain variable region (VL), the C-3 can be a light chain constant region (CL), the V-4 can be a VL, and the C-4 can be a CL. In another aspect, the V-1 can be a VH, the C-1 can be a CH1, the V-2 can be a VL, the C-2 can be a CL, the V-3 can be a VL, the C-3 can be a CL, the V-4 can be a VH, and the C-4 can be a CH1. In a further embodiment, the V-1 can be a VH, the C-1 can be a CH1, the V-2 can be a VH, the C-2 can be a CL, the V-3 can be a VL, the C-3 can be a CL, the V-4 can be a VL, and the C-4 can be a CH1. In another embodiment, the V-1 can be a VH, the C-1 can be a CL, the V-2 can be a VH, the C-2 can be a CH1, the V-3 can be a VL, the C-3 can be a CH1, the V-4 can be a VL, and the C-4 can be a CL. In another embodiment, the V-1 can be a VL, the C-1 can be a CL, the V-2 can be a VL, the C-2 can be a CL, the V-3 can be a VH, the C-3 can be a CH1, the V-4 can be a VH, and the C-4 can be a CH1. In still another aspect, the V-1 can be a VL, the C-1 can be a CL, the V-2 can be a VH, the C-2 can be a CH1, the V-3 can be a VH, the C-3 can be a CH1, the V-4 can be a VL, and the C-4 can be a CL. In another embodiment, the V-1 can be a VL, the C-1 can be a CH1, the V-2 can be a VH, the C-2 can be a CH1, the V-3 can be a VH, the C-3 can be a CL, the V-4 can be a VL, and the C-4 can be a CL. In still another embodiment, the V-1 can be a VL, the C-1 can be a CH1, the V-2 can be a VL, the C-2 can be a CH1, the V-3 can be a VH, the C-3 can be a CL, the V-4 can be a VH, and the C-4 can be a CL. In a further embodiment, the V-1 can be a VH, the C-1 can be a CH1, the V-2 can be a VL, the C-2 can be a CH1, the V-3 can be a VL, the C-3 can be a CL, the V-4 can be a VH, and the C-4 can be a CL. In one further aspect, the V-1 can be a VL, the C-1 can be a CH1, the V-2 can be a VH, the C-2 can be a CL, the V-3 can be a VH, the C-3 can be a CL, the V-4 can be a VL, and the C-4 can be a CH1.

In some embodiments of the V-C-Fc-V-C antibodies described above and below, the first polypeptide chain can comprise a linker between the CH3-1 and the V-2 and the second polypeptide chain can comprise a linker between the CH3-2 and the V-4. In the invention, either the linker between CH3-1 and V-2 or the linker between CH3-2 and V-4 comprises a cleavage site for a protease, which can be a furin, a matrix metalloproteinase, or a protease expressed by a cancer cell. The linkers between the CH3-1 and the V-2 and between the CH3-2 and the V-4 each can be, for example, from 5 to 35 or from 5 to 30 amino acids long.

In the V-C-Fc-V-C antibodies described above and below, the CH3-1 and the CH3-2 can have different amino acid sequences and can comprise charge pair substitutions. The CH3-1 can, for example, comprise the charge pair substitutions K409D or K409E and K392D or K392E, and the CH3-2 can, for example, comprise the charge pair substitutions D399K or D399R and D356K or D356R. Alternatively, the CH3-2 can, for example, comprise the charge pair substitutions K409D or K409E and K392D or K392E, and the CH3-1 can, for example, comprise the charge pair substitutions D399K or D399R and D356K or D356R.

In the V-C-Fc-V-C-antibodies described above and below, the V-1 and the V-3 can bind to an immune effector cell when they are part of an IgG and/or scFv antibody, and the V-2 and the V-4 can bind to a target cell when they are part of an IgG and/or scFv antibody. Alternately, the V-2 and the V-4 can bind to an immune effector cell when they are part of an IgG and/or scFv antibody, and the V-1 and the V-3 can bind to a target cell when they are part of an IgG and/or scFv antibody. The immune effector cell is a T cell, and the effector cell molecule is a human CD3 epsilon chain (CD3ε), and the target cell is a cancer cell.

In some embodiments of the V-C-Fc-V-C antibody described above and below, the C-1 to C4, the hinge regions, and the CH2-1, CH2-2, CH3-1, and CH3-2 can be human IgG constant regions or human lambda or kappa constant regions, which human sequences comprise not more than 15 insertions, deletions, or substitutions of a single amino acid per 100 amino acids relative to a naturally-occurring human amino acid sequence. The human IgG constant regions can be human IgG1, IgG2, or IgG4 constant regions. In another aspect, the first and second polypeptide chains can comprise one or more alterations that reduce Fc gamma receptor (FcγR) binding, such as L234A, L235A, and/or any substitution at N297. In a further aspect, the first and second polypeptide chains can comprise one or more alterations that enhance(s) ADCC, such as an insertion of the amino acid sequence of any one of SEQ ID NOs:13-24 between positions 384 and 385 according to the EU numbering system as shown in Table 2 in CH3-1 and/or CH3-2.

In a further embodiment, herein is described a V-C-Fc-V-C antibody comprising a first polypeptide chain having the formula V-1---C-1---hinge---CH2-1---CH3-1---L-1---V-2---C-2 and a second polypeptide chain having the formula V-3---C-3---hinge---CH2-2---CH3-2---L-2---V-4---C-4; wherein the V-1, V-2, V-3, and V-4 are immunoglobulin variable regions having different amino acid sequences; wherein either the V-1 or the V-3 is a VH, and if the V-1 is a VH, then the V-3 is a VL, and if the V-3 is a VH, then the V-1 is a VL; wherein either the V-2 or the V-4 is a VH, and if the V-2 is a VH, then the V-4 is a VL, and if the V-4 is a VH, then the V-2 is a VL; wherein the L-1 and L-2 are linkers; wherein either the C-1 or the C-3 is a CH1, and if the C-1 is a CH1, then the C-3 is a CL, and if the C-3 is a CH1, then the C-1 is a CL; wherein either the C-2 or the C-4 is a CH1, and if the C-2 is a CH1, then the C-4 is a CL, and if the C-4 is a CH1, then the C-2 is a CL; and wherein the antibody can bind to a target cell, which can be a cancer cell, an infected cell, or a cell mediating an autoimmune or fibrotic disease, and to an immune effector cell. Alternatively, herein is described an antibody comprising a first polypeptide chain having the following formula: V-1---L-3---C-1---L-4---hinge---CH2-1---CH3-1---L-1---V-2---L-5--C-2 and a second polypeptide chain having the following formula: V-3-L6---C-3---L7---hinge---CH2-2---CH3-2---L-2---V-4---L-8---C-4, wherein the V-1, V-2, V-3, and V-4 are immunoglobulin variable regions having different amino acid sequences, wherein either the V-1 or the V-3 is a VH, and if the V-1 is a VH, then the V-3 is a VL, and if the V-3 is a VH, then the V-1 is a VL, wherein either the V-2 or the V-4 is a VH, and if the V-2 is a VH, then the V-4 is a VL, and if the V-4 is a VH, then the V-2 is a VL, wherein the L-1, L-2, L-3, L-4, L-5, L-6, L-7, and L-8 are linkers and L-3, L-4, L-5, L-6, L-7, and L-8 can be present or absent, wherein either the C-1 or the C-3 is a CH1, and if the C-1 is a CH1, then the C-3 is a CL, and if the C-3 is a CH1, then the C-1 is a CL, wherein either the C-2 or the C-4 is a CH1, and if the C-2 is a CH1, then the C-4 is a CL, and if the C-4 is a CH1, then the C-2 is a CL, and wherein the antibody binds to a cancer cell and to an immune effector cell. The first and the second polypeptide chains can each comprise a charge pair substitution. For example, the CH3-1 can comprise the charge pair substitutions K409D or K409E and K392D or K392E, and the CH3-2 can comprise the charge pair substitutions D399K or D399R and E356K or E356R. Alternatively, the CH3-2 can comprise the charge pair substitutions K409D or K409E and K392D or K392E, and the CH3-1 can comprise the charge pair substitutions D399K or D399R and D356K or D356R. Alternatively or in addition, the CH3-1 and the CH3-2 can comprise other charge pair substitutions. In another aspect, the first and second polypeptide chains can comprise one or more alterations that inhibit FcγR binding, such as, for example, L234A, L235A, and any substitution at N297. In some embodiments, the immune effector cell can be a T cell, and the antibody can bind to human CD3ε. In some embodiments, the target cell is a cancer cell. In such embodiments, the V-1 and the V-3 may bind to the cancer cell when they are part of an IgG and/or an scFv antibody, and the V-2 and the V-4 may bind to the immune effector cell when they are part of an IgG and/or scFv antibody. Alternatively the V-2 and the V-4 may bind to the cancer cell when they are part of an IgG and/or an scFv antibody and the V-1 and the V-3 may bind to the immune effector cell when they are part of an IgG and/or scFv antibody. In some embodiments, the L-1 can comprise a protease cleavage site while the L-2 does not, or vice versa.

In some of the embodiments of a V-C-Fc-V-C antibody described above and/or below, the antibody can comprise the amino acid sequences of SEQ ID NOs: 1 and 3, SEQ ID NOs: 5 and 7, SEQ ID NOs: 9 and 11, SEQ ID NOs: 46 and 48, SEQ ID NOs:11 and 62, SEQ ID NOs:11 and 64, or SEQ ID NOs:67 and 69.

In another aspect, herein is described a pharmaceutical composition comprising any of the V-C-Fc-V-C antibodies described above and/or below.

Also provided herein are one or more nucleic acid(s) encoding any of the V-C-Fc-V-C antibodies, or portions thereof, described above and/or below. Such nucleic acids can include the nucleotide sequences of SEQ ID NOs:2 and 4, SEQ ID NOs:6 and 8, SEQ ID NOs:10 and 12, SEQ ID NOs:47 and 49, SEQ ID NOs:12 and 63, SEQ ID NOs:12 and 65, or SEQ ID NOs:68 and 70. Also described are one or more vector(s) containing such nucleic acid(s) and a host cell containing such nucleic acids or vectors. Also described herein is a method of making an antibody comprising culturing such a host cell under conditions such that the nucleic acid(s) is(are) expressed, and recovering the antibody from the cell mass or the culture medium.

In further embodiments, herein are described antibodies for use in a method of treatment comprising administering to a cancer patient a therapeutically effect dose of any of the V-C-Fc-V-C antibodies described above or below. The cancer can be a hematologic malignancy. Chemotherapy, a non-chemotherapeutic anti-neoplastic agent, or radiation may have been administered before, after, or concurrently with the antibody. In some embodiments, the antibody can bind to FOLR1 or HER2 and CD3ε.

In another aspect, herein are described antibodies for use in a method of treatment comprising administering to a patient having an infectious disease any of the V-C-Fc-V-C antibodies described above and/or below. In a further aspect, herein are provided antibodies for use in a method of treatment comprising administering to a patient having asthma, systemic lupus erythematosus, or a fibrotic disease any of the V-C-Fc-V-C antibodies described above and/or below. In still another aspect, herein is described the use of any of the V-C-Fc-V-C antibodies described above and/or below as a medicament for treating a cancer, an infectious disease, an autoimmune disease, asthma, or a fibrotic disease. Further described herein is use of any of the V-C-Fc-V-C antibodies described above and/or below in the manufacture of a medicament for treating a cancer, an infectious disease, an autoimmune disease, asthma, or a fibrotic disease.

## BRIEF DESCRIPTION OF THE FIGURES

**Figure** 1: Diagram of a V-C-Fc-V-C antibody. The various regions are shown as follows: oval filled with heavy horizontal lines, immunoglobulin variable region (labeled V-1 through V-4); unfilled rectangle, immunoglobulin constant region (labeled C-1 through C-4), which can be either a CH1 or a CL region; oval filled with diagonal lines, CH2 region (labeled CH2-1 and CH2-2); oval filled with windowpane checks, CH3 region (labeled CH3-1 and CH3-2); vertical line between the most N-terminal immunoglobulin constant region and the CH2 region, hinge region; horizontal or nearly horizontal line, disulfide bond; vertical line between the CH3 region and the following immunoglobulin variable region, linker; and short vertical line following one of the C-terminal immunoglobulin constant regions, a portion of a hinge region. The position of the Fc region is indicated.

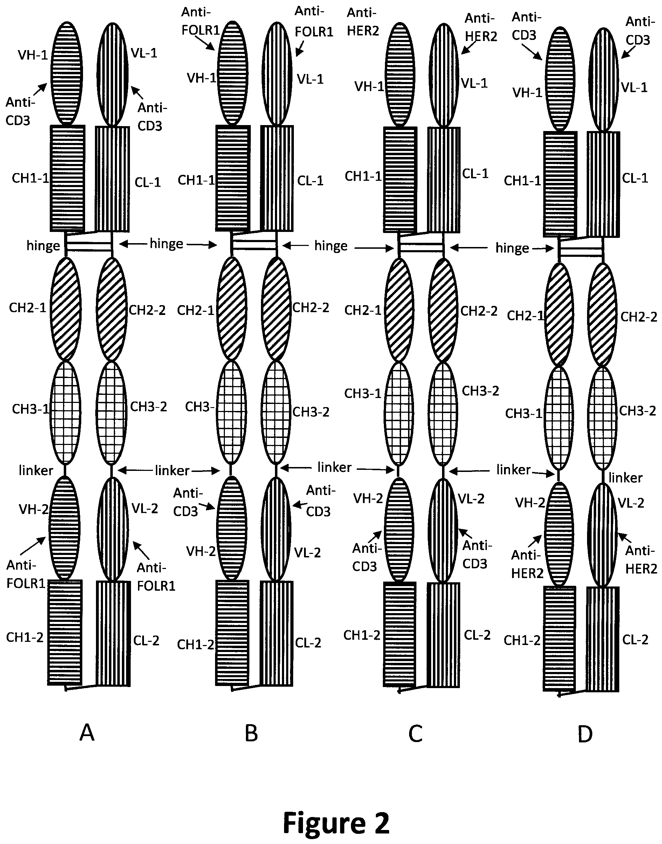

**Figure 2:** Diagrams of particular embodiments of V-C-Fc-V-C antibodies. In panels A, B, C, and D, respectively, are diagrammed V-C-Fc-V-C antibodies called aCD3-Fc-aFOLR1, aFOLR1-Fc-aCD3, aHER2-Fc-aCD3, and aCD3-Fc-aHER2. The construction of nucleic acids encoding these and other antibodies are described in Example 1. Amino acid sequences of the two polypeptide chains that constitute each of these are provided in SEQ ID NOs: 1 and 3 (aFOLR1-Fc-aCD3), SEQ ID NOs: 5 and 7 (aCD3-Fc-aFOLR1), SEQ ID NOs: 9 and 11 (aHER2-Fc-aCD3), and SEQ ID NOs:46 and 48 (aCD3-Fc-aHER2). The various regions are shown as follows: oval filled with heavy horizontal lines, VH region; rectangle filled with heavy horizontal lines, CH1 region; vertical line between the CH1 or CL region and the CH2 region, hinge region; horizontal or nearly horizontal line, disulfide bond; oval filled with diagonal lines, CH2 region; oval filled with windowpane checks, CH3 region; vertical line between the CH3 region and the following immunoglobulin variable region, linker; oval filled with heavy vertical lines, VL region; rectangle filled with heavy vertical lines, CL region; and vertical line following the most carboxyterminal CH1 region, a portion of the hinge.

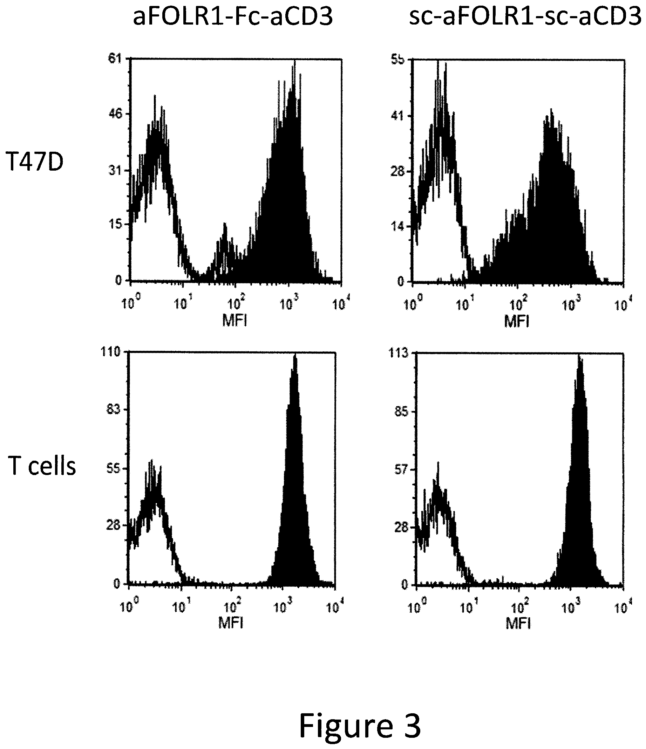

**Figure 3:** Binding of aFOLR1-Fc-aCD3 or sc-aFOLR1-sc-aCD3 to cells expressing FOLR1 or CD3. The binding of aFOLR1-Fc-aCD3 and sc-aFOLR1-sc-aCD3 to T47D tumor cells, which express FOLR1, and T cells, which express CD3, was analyzed by fluorescence activated cell sorting (FACS) as described in Example 2. The x axes show the intensity of fluorescence detected (MFI), and the y axes show the number of cells. Data from T47D cells and T cells are shown in the top and bottom two panels, respectively, as indicated. Data from aFOLR1-Fc-aCD3 and sc-aFOLR1-sc-aCD3 are shown in the left and right two panels, respectively, as indicated.

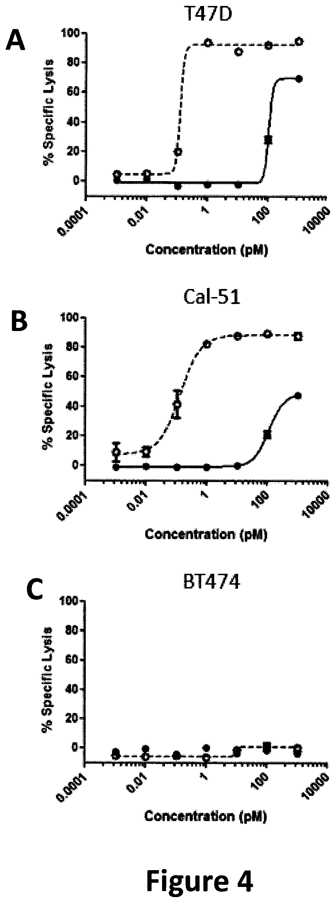

**Figure 4:** T cell dependent cell cytolysis induced by aFOLR1-Fc-aCD3 or sc-aFOLR1-sc-aCD3. Assays were performed as described in Example 2 using the following three different target cell lines: panel A, T47D, which expresses about 101,000 molecules of FOLR1 on the surface of each cell; panel B, Cal-51, which expresses about 148,000 molecules of FOLR1 on the surface of each cell; and panel C, BT474, which expresses no detectable FOLRI on the surface of the cells. Filled circles represent data from aFOLR1-Fc-aCD3 (which is described in Example 1), and open circles represent data from sc-aFOLR1-sc-aCD3, which is described in Example 2. The x axis indicates the concentration of protein used, and the y axis indicates the percent of target cells lysed.

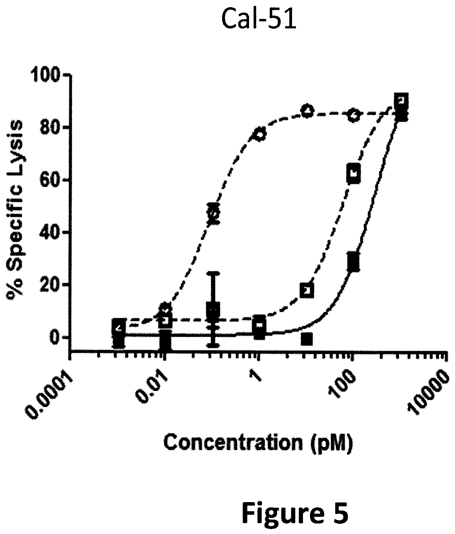

**Figure 5:** T cell dependent cell cytolysis induced by aFOLR1-Fc-aCD3, aCD3-Fc-aFOLR1, or sc-aFOLR1-sc-aCD3. Assays were performed as described in Example 2 using Cal-51 cells, as indicated. Symbols indicate data from assays containing the following bispecific molecules: open circles with dashed line, sc-aFOLR1-sc-aCD3; filled squares with solid line, aCD3-Fc-aFOLR1; and open squares with dashed line, aFOLR1-Fc-aCD3.

The x axis indicates the concentration of protein used, and the y axis indicates the percent of target cells lysed.

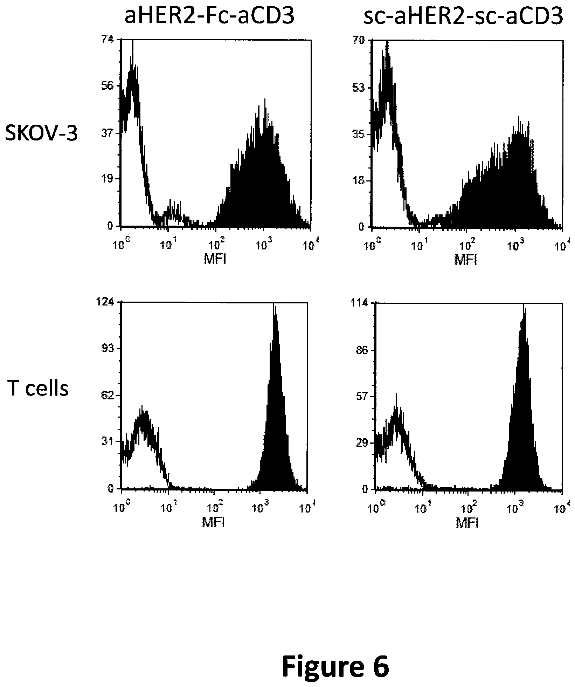

**Figure 6:** Binding of aHER2-Fc-aCD3 or sc-HER2-sc-aCD3 to cells expressing HER2 or CD3. The binding of aHER2-Fc-aCD3 and sc-aHER2-sc-aCD3 to SKOV-3 tumor cells, which express HER2, and T cells, which express CD3, was analyzed by FACS as described in Example 3. Data from SKOV-3 cells and T cells are shown in the top and bottom two panels, respectively, as indicated. Data from aHER2-Fc-aCD3 and sc-aHER2-sc-aCD3 are shown in the left and right two panels, respectively, as indicated. The x axes show the intensity of fluorescence detected (MFI), and the y axes show the number of cells.

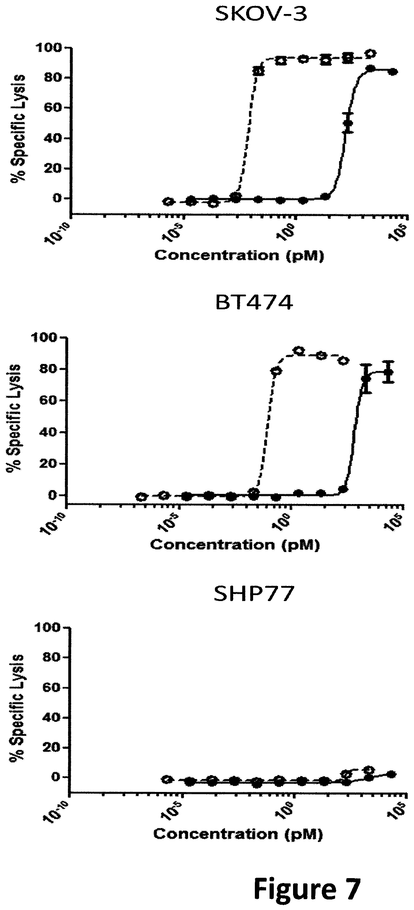

**Figure 7:** T cell dependent cell cytolysis induced by aHER2-Fc-aCD3 or sc-aHER2-sc-aCD3. Assays were performed as described in Example 3 using the following three different target cell lines: top panel, SKOV-3, which expresses about 530,000 molecules of HER2 on the surface of each cell; middle panel, BT474, which expresses about 300,000 molecules of HER2 on the surface of each cell; and bottom panel, SHP77, which expresses no detectable HER2 on the surface of the cells. Filled circles represent data from aHER2-Fc-aCD3, which is described in Example 1, and open circles represent data from the sc-aHER2-sc-aCD3, which is described in Example 3. The x axis indicates the concentration (pM) of protein used, and the y axis indicates the percent of target cells lysed.

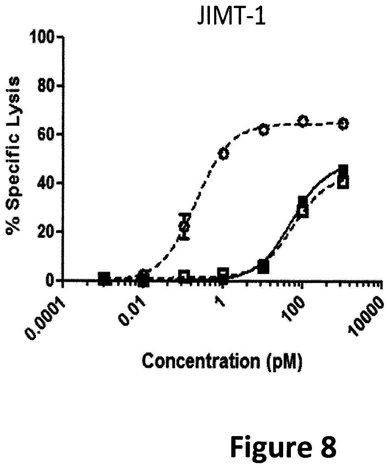

**Figure 8:** T cell dependent cell cytolysis induced by aHER2-Fc-aCD3, aCD3-Fc-aHER2, or sc-aHER2-sc-aCD3. Assays were performed as described in Example 3, except that JIMT-1 cells, which express about 181,000 molecules of HER2 per cell, were used as target cells. Symbols indicate data from assays containing the following bispecific molecules: open circles with dashed line, sc-aHER2-sc-aCD3; filled squares with solid line, aCD3-Fc-aHER2; and open squares with dashed line, aHER2-Fc-aCD3. The x axis indicates the concentration of protein used, and the y axis indicates the percent of target cells lysed.

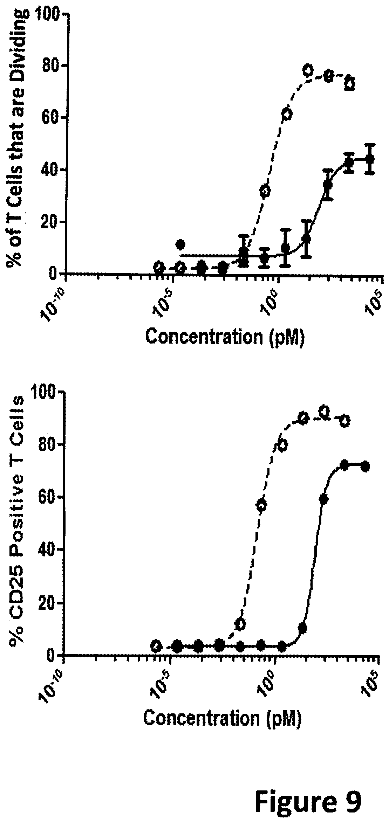

**Figure 9:** Activation of T cells by aHER2-Fc-aCD3 or sc-aHER2-sc-aCD3. In the top panel the y axis shows the percent of T cells in a population incubated with either aHER2-Fc-aCD3 (filled circles) or sc-HER2-sc-aCD3 (open circles) that are dividing. In the bottom panel the y axis shows the percent of cells expressing CD25 in a population of T cells incubated with either aHER2-Fc-aCD3 (filled circles) or sc-aHER2-sc-aCD3 (open circles). The x axis shows the concentration of protein used (pM). Methods are described in Example 3.

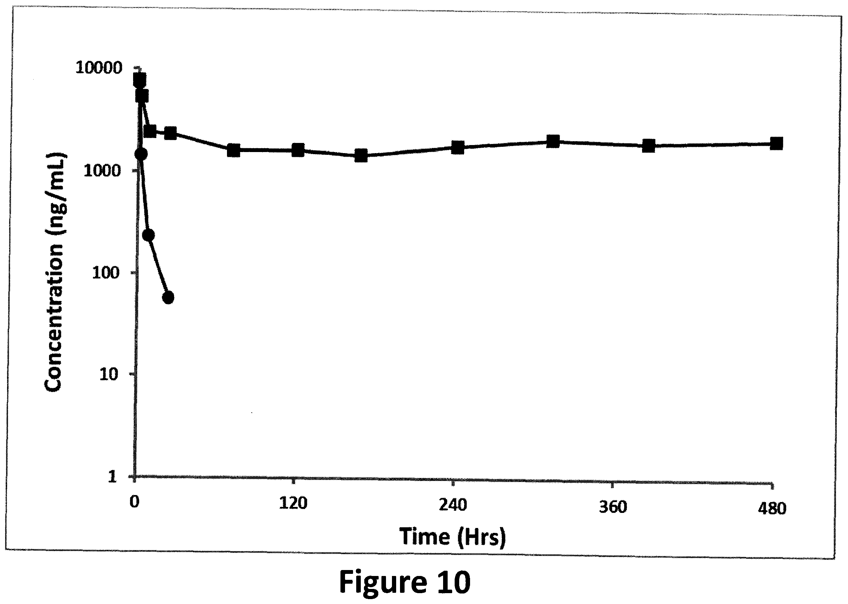

**Figure 10:** Pharmacokinetics following intravenous injection. Two different bispecific antibodies, a V-C-Fc-V-C (aHER2-Fc-aCD3) and a single chain antibody (sc-aHER2-sc-aCD3), were injected intravenously into mice, and concentrations were assessed over time as described in Example 4. Symbols indicate data from animals injected with sc-aHER2-sc-aCD3 (filled circles), or aHER2-Fc-aCD3 (filed squares).

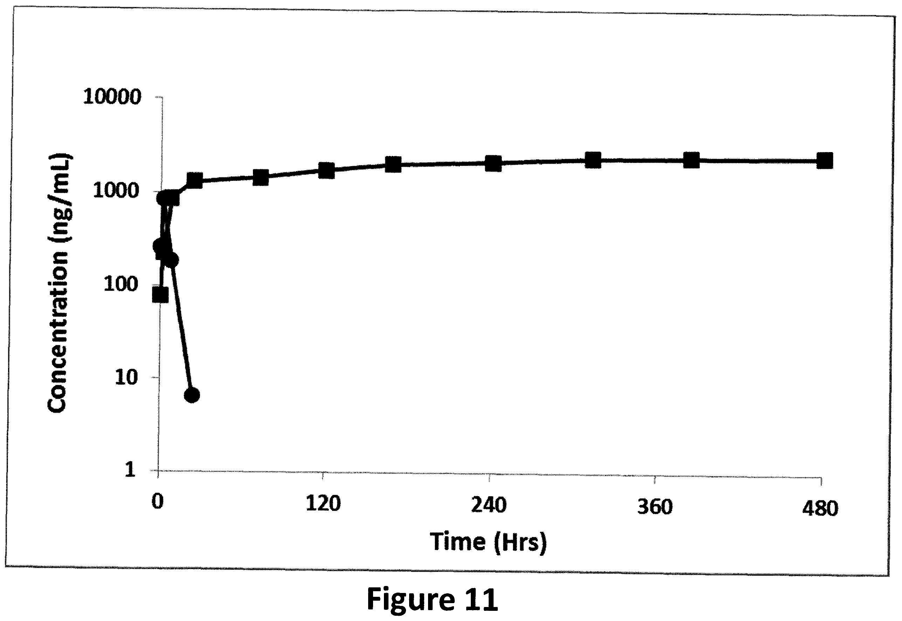

**Figure 11:** Pharmacokinetics following subcutaneous injection. Two different bispecific antibodies, a V-C-Fc-V-C (aHER2-Fc-aCD3) and a single chain antibody (sc-aHER2-sc-aCD3), were injected intravenously into mice, and concentrations were assessed over time as described in Example 4. Symbols indicate data from animals injected with sc-aHER2-sc-aCD3 (filled circles), or aHER2-Fc-aCD3 (filed squares).

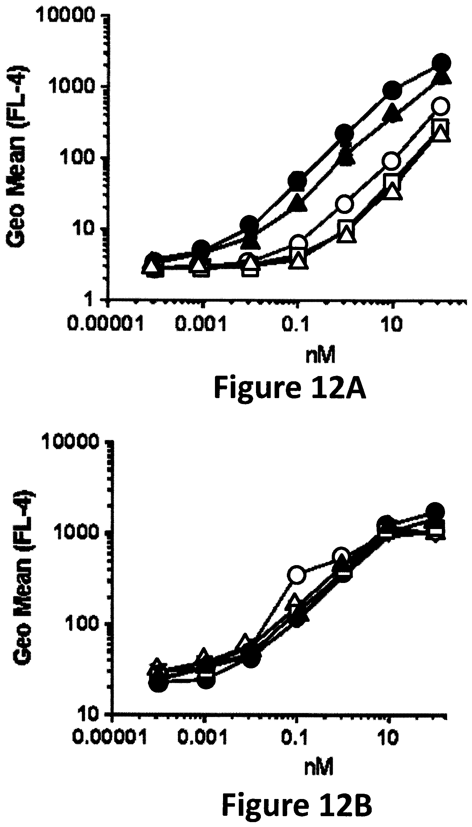

**Figure 12A:** Binding of V-C-Fc-V-Cs with and without protease cleavage sites to T cells. Methods are described in Example 2. The x axis shows the concentration (nM) of protein used in the assay, and the y axis shows the geometric mean of the fluorescence detected in the FACS at that concentration. Symbols signify data from assays containing the following proteins: filled circles, the positive control heterodimeric antibody described in Example 5, comprising SEQ ID NOs:60 and 61; unfilled circles and unfilled squares, aHer2-Fc-aCD3, comprising SEQ ID NOs:9 and 11; filled triangles, aHer2-Fc-Fur-aCD3, comprising SEQ ID NOs: 11 and 62; and unfilled triangles, aHer2-Fc-MMP2-aCD3, comprising SEQ ID NOs: 11 and 64.

**Figure 12B:** Binding of V-C-Fc-V-Cs with and without protease cleavage sites to T47D cells. Methods are described in Example 2. The x axis shows the concentration of protein used in the assay, and the y axis shows the geometric mean of the fluorescence detected in the FACS at that concentration. Symbols signify as stated in the description of Figure 12A.

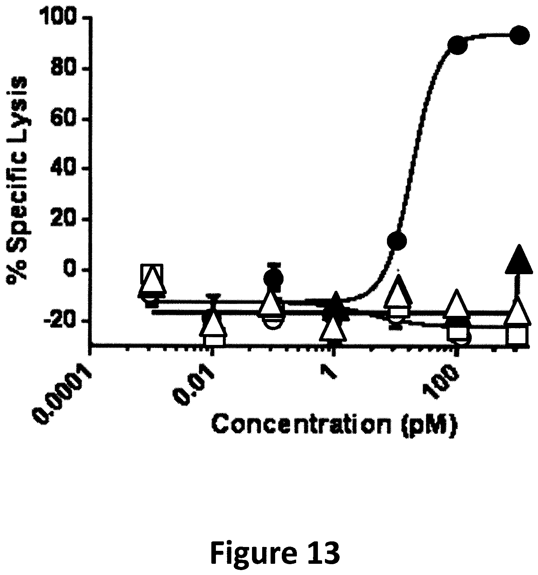

**Figure 13:** Cytolysis of of T47D cells in the presence of T cells and V-C-Fc-V-Cs with and without protease cleavage sites or control proteins. Cytolysis assays using T47D cells as target cells are described in Example 2. The x axis shows the concentration of protein used and the y axis shows the percent of target cells lysed. Symbols signify as stated in the description of Figure 12A.

## BRIEF DESCRIPTION OF THE SEQUENCE LISTINGS

| SEQ ID NO | Description |
|---|---|
| SEQ ID NO:1 | Amino acid sequence of one polypeptide chain of aFOLR1-Fc-aCD3 with signal sequence |
| SEQ ID NO:2 | Nucleic acid sequence encoding SEQ ID NO:1 |
| SEQ ID NO:3 | Amino acid sequence of another polypeptide chain of aFOLR1-Fc-aCD3 with signal sequence |

(continued)

| SEQ ID NO | Description |
|---|---|
| SEQ ID NO:4 | Nucleic acid sequence encoding SEQ ID NO:3 |
| SEQ ID NO:5 | Amino acid sequence of one polypeptide chain of aCD3-Fc-aFOLR1 with signal sequence |
| SEQ ID NO:6 | Nucleic acid sequence encoding SEQ ID NO:5 |
| SEQ ID NO:7 | Amino acid sequence of another polypeptide chain of aCD3-Fc-aFOLR1 with signal sequence |
| SEQ ID NO:8 | Nucleic acid sequence encoding SEQ ID NO:7 |
| SEQ ID NO:9 | Amino acid sequence of one polypeptide chain of aHER2-Fc-aCD3 with signal sequence |
| SEQ ID NO:10 | Nucleic acid sequence encoding SEQ ID NO:9 |
| SEQ ID NO:11 | Amino acid sequence of a polypeptide chain of aHER2-Fc-aCD3, aHER2-Fc-Fur-aCD3, or aHER2-Fc-MMP2-aCD3, with signal sequence |
| SEQ ID NO:12 | Nucleic acid sequence encoding SEQ ID NO:11 |
| SEQ ID NO:13 | Amino acid sequence of peptide that extends half life of an Fc region |
| SEQ ID NO:14 | Amino acid sequence of peptide that extends half life of an Fc region |
| SEQ ID NO:15 | Amino acid sequence of peptide that extends half life of an Fc region |
| SEQ ID NO:16 | Amino acid sequence of peptide that extends half life of an Fc region |
| SEQ ID NO:17 | Amino acid sequence of peptide that extends half life of an Fc region |
| SEQ ID NO:18 | Amino acid sequence of peptide that extends half life of an Fc region |
| SEQ ID NO:19 | Amino acid sequence of peptide that extends half life of an Fc region |
| SEQ ID NO:20 | Amino acid sequence of peptide that extends half life of an Fc region |
| SEQ ID NO:21 | Amino acid sequence of peptide that extends half life of an Fc region |
| SEQ ID NO:22 | Amino acid sequence of peptide that extends half life of an Fc region |
| SEQ ID NO:23 | Amino acid sequence of peptide that extends half life of an Fc region |
| SEQ ID NO:24 | Amino acid sequence of peptide that extends half life of an Fc region |
| SEQ ID NO:25 | Amino acid sequence of human fibronectin 3 domain engineered to bind serum albumin |
| SEQ ID NO:26 | Amino acid sequence of human IgG1 Fc polypeptide chain |
| SEQ ID NO:27 | Amino acid sequence of human IgG2 Fc polypeptide chain |
| SEQ ID NO:28 | Amino acid sequence of human IgG3 Fc polypeptide chain |
| SEQ ID NO:29 | Amino acid sequence of human IgG4 Fc polypeptide chain |
| SEQ ID NO:30 | Amino acid sequence preceding heavy chain CDR1 |
| SEQ ID NO:31 | Amino acid sequence preceding heavy chain CDR2 |
| SEQ ID NO:32 | Amino acid sequence following heavy chain CDR3 |
| SEQ ID NO:33 | Amino acid sequence following light chain CDR3 |
| SEQ ID NO:34 | Amino acid sequence of a linker |
| SEQ ID NO:35 | Amino acid sequence of a linker |
| SEQ ID NO:36 | Amino acid sequence of a linker |
| SEQ ID NO:37 | Amino acid sequence of a linker |
| SEQ ID NO:38 | Amino acid sequence of a linker |
| SEQ ID NO:39 | Mature amino acid sequence of CD3ε of *Homo sapiens* |
| SEQ ID NO:40 | Mature amino acid sequence of CD3ε of *Macaca fascicularis* |

(continued)

| SEQ ID NO | Description |
|---|---|
| SEQ ID NO:41 | Amino acid sequence of a portion of an epitope on human CD3ε |
| SEQ ID NO:42 | Amino acid sequence of sc-aHER2-sc-aCD3 with signal sequence |
| SEQ ID NO:43 | Nucleic acid sequence encoding SEQ ID NO:42 |
| SEQ ID NO:44 | Amino acid sequence of sc-aFOLR1-sc-aCD3 with signal sequence |
| SEQ ID NO:45 | Nucleic acid sequence encoding SEQ ID NO:44 |
| SEQ ID NO:46 | Amino acid sequence of one polypeptide chain of aCD3-Fc-aHER2 with signal sequence |
| SEQ ID NO:47 | Nucleic acid sequence encoding SEQ ID NO:46 |
| SEQ ID NO:48 | Amino acid sequence of another polypeptide chain of aCD3-Fc-aHER2 with signal sequence |
| SEQ ID NO:49 | Nucleic acid sequence encoding SEQ ID NO:48 |
| SEQ ID NO:50 | Amino acid sequence of a linker |
| SEQ ID NO:51 | Amino acid sequence of a linker that can be cleaved by a metalloproteinase |
| SEQ ID NO:52 | Amino acid sequence of a linker that can be cleaved by a furin |
| SEQ ID NO:53 | Amino acid sequence of a linker that can be cleaved by a protease |
| SEQ ID NO:54 | Amino acid sequence of a linker that can be cleaved by a protease |
| SEQ ID NO:55 | Amino acid sequence of a linker that can be cleaved by a protease |
| SEQ ID NO:56 | Amino acid sequence of a linker that can be cleaved by a protease |
| SEQ ID NO:57 | Amino acid sequence of a linker that can be cleaved by a protease |
| SEQ ID NO:58 | Amino acid sequence of a linker that can be cleaved by a protease |
| SEQ ID NO:59 | Amino acid sequence that can be cleaved by matrix metalloproteinase 9 (MMP9) |
| SEQ ID NO:60 | Amino acid sequence of one polypeptide chain of aCD3/HER2 heterodimer (P136797.3) |
| SEQ ID NO:61 | Amino acid sequence of the other polypeptide chain of aCD3/HER2 heterodimer (P136797.3) |
| SEQ ID NO:62 | Amino acid sequence a second polypeptide chain of aHER2-Fc-Fur-aCD3 (PL-35586) with signal sequence |
| SEQ ID NO:63 | Nucleic acid sequence encoding SEQ ID NO:62 |
| SEQ ID NO:64 | Amino acid sequence a second polypeptide chain of aHER2-Fc-MMP2-aCD3 (PL-35589) with signal sequence |
| SEQ ID NO:65 | Nucleic acid sequence encoding SEQ ID NO:64 |
| SEQ ID NO:66 | Amino acid sequence of an MMP2 cleavage site |
| SEQ ID NO:67 | Amino acid sequence of an anti-CD3 VH region |
| SEQ ID NO:68 | Nucleic acid sequence encoding SEQ ID NO:67 |
| SEQ ID NO:69 | Amino acid sequence of an anti-CD3 VL region |
| SEQ ID NO:70 | Nucleic acid sequence encoding SEQ ID NO:69 |
| SEQ ID NO:71 | Amino acid sequence of a meprin α or β cleavage site |
| SEQ ID NO:72 | Amino acid sequence of a meprin α or β cleavage site |
| SEQ ID NO:73 | Amino acid sequence of a meprin α or β cleavage site |
| SEQ ID NO:74 | Amino acid sequence of a meprin α or β cleavage site |
| SEQ ID NO:75 | Amino acid sequence of a urokinase-type plasminogen activator (u-PA) cleavage site |
| SEQ ID NO:76 | Amino acid sequence of a u-PA cleavage site |

(continued)

| SEQ ID NO | Description |
|---|---|
| SEQ ID NO:77 | Amino acid sequence of a u-PA cleavage site |
| SEQ ID NO:78 | Amino acid sequence of a u-PA cleavage site |
| SEQ ID NO:79 | Amino acid sequence of a u-PA cleavage site |
| SEQ ID NO:80 | Amino acid sequence of a u-PA cleavage site |
| SEQ ID NO:81 | Amino acid sequence of a u-PA cleavage site |
| SEQ ID NO:82 | Amino acid sequence of a tissue plasminogen activator (tPA) cleavage site |
| SEQ ID NO:83 | Amino acid sequence of a cathepsin B cleavage site |
| SEQ ID NO:84 | Amino acid sequence of a cathepsin B cleavage site |
| SEQ ID NO:85 | Amino acid sequence of a cathepsin B cleavage site |
| SEQ ID NO:86 | Amino acid sequence of a cathepsin B cleavage site |
| SEQ ID NO:87 | Amino acid sequence of a cathepsin B cleavage site |
| SEQ ID NO:88 | Amino acid sequence of a cathepsin B cleavage site |
| SEQ ID NO:89 | Amino acid sequence of a cathepsin B cleavage site |
| SEQ ID NO:90 | Amino acid sequence of a cathepsin B cleavage site |
| SEQ ID NO:91 | Amino acid sequence of a cathepsin B cleavage site |
| SEQ ID NO:92 | Amino acid sequence of a cathepsin B cleavage site |
| SEQ ID NO:93 | Amino acid sequence of a cathepsin B cleavage site |
| SEQ ID NO:94 | Amino acid sequence of a cathepsin B cleavage site |
| SEQ ID NO:95 | Amino acid sequence of a cathepsin B cleavage site |
| SEQ ID NO:96 | Amino acid sequence of a cathepsin B cleavage site |
| SEQ ID NO:97 | Amino acid sequence of a furin cleavage site |
| SEQ ID NO:98 | Amino acid sequence of a furin cleavage site |
| SEQ ID NO:99 | Amino acid sequcne of an MMP2 cleavage site |
| SEQ ID NO:100 | Amino acid sequcne of an MMP2 cleavage site |
| SEQ ID NO:101 | Amino acid sequence of a cathepsin B cleavage site |

## DETAILED DESCRIPTION

Described herein is a new format for a bispecific antibody. It is a heterodimeric molecule containing two different polypeptide chains. Each polypeptide chain comprises an Fc polypeptide chain, which is between two different segments, each containing a heavy or light chain variable (VH or VL) region and either a heavy chain constant region 1 (CH1) or a light chain constant region (CL). This format is diagramed in Figure 1. Together, the two chains contain two different binding sites, each of which comprises a VH and a VL region, and each of which binds to a different molecule, optionally a protein. One of the proteins is expressed on the surface of an immune effector cell, specifically a T cell. Also disclosed are proteins expressed on other immune effector cells such as an NK cell, a macrophage, or a neutrophil. The other protein is expressed on the surface of a target cell, specifically a cancer cell. Also disclosed as target cell is a cell infected by a pathogen such as a virus, or a cell that mediates a fibrotic, autoimmune, or inflammatory disease. Since a V-C-Fc-V-C antibody, as described herein, has only one binding site for each of the molecules or proteins it binds to (*i.e.,* it binds "monovalently" to each molecule or protein), its binding cannot oligomerize the molecules or proteins it binds to on a cell surface in some situations, *e.g.,* if it is bound to only one of its target proteins. For example, if it binds to CD3 on the surface of a T cell, CD3 will not be oligomerized on the T cell surface. Oligomerization of CD3 can cause a generalized activation of a T cell, which can be undesirable. The V-C-Fc-V-C antibody described herein can tether an immune effector cell to a target cell, forming a close physical association between the cells, thereby eliciting a specific cytolytic activity

8

against the target cell rather than a generalized inflammatory response. For example, when an immune effector cell protein, for example CD3 on a T cell, and a target cell protein are simultaneously engaged by a V-C-Fc-V-C, the CD3 can be oligomerized on the T cell, which is thereby activated to kill the target cell to which it is tethered. The mechanism of action may be similar to that explored in detail for other bispecific antibodies. *See, e.g.,* Haas et al. (2009), Immunobiology 214(6): 441-453. Further, the V-C-Fc-V-C antibodies can comprise at least one, optionally two, half life-extending moieties. Thus, they can have favorable pharmacokinetic properties and can be relatively easy to manufacture since they contain only two different polypeptide chains.

## *Definitions*

Antibodies comprise various domains or regions. For example, an antibody in the IgG format found in nature comprises four polypeptide chains, two heavy chains and two light chains. Each heavy chain comprises a VH region followed by a first heavy chain constant (CH1) region, a hinge region, a second heavy chain constant (CH2) region, and a third heavy chain constant (CH3) region. Each light chain comprises a light chain variable (VL) region and a light chain constant (CL) region. These regions are described in, *e.g.,* Carayannopoulos and Capra, Immunoglobulins: Structure and Function, pp. 283-314, in Fundamental Immunology, 3rd Edition, Paul, ed., Raven Press, New York, 1993.

An **"antibody,"** as meant herein, is a protein containing at least one VH or VL region, in many cases a VH and a VL region. Thus, the term "antibody" encompasses molecules having a variety of formats, including single chain Fv antibodies (scFv, which contain VH and VL regions joined by a linker), Fab, F(ab)$_2$', Fab', scFv:Fc antibodies (as described in Carayannopoulos and Capra, Ch. 9 in FUNDAMENTAL IMMUNOLOGY, 3rd ed., Paul, ed., Raven Press, New York, 1993, pp. 284-286) or full length antibodies containing two full length heavy and two full length light chains, such as naturally-occurring IgG antibodies found in mammals. *Id.* Such IgG antibodies can be of the IgG1, IgG2, IgG3, or IgG4 isotype and can be human antibodies. Further, the term "antibody" includes dimeric antibodies containing two heavy chains and no light chains such as the naturally-occurring antibodies found in camels and other dromedary species and sharks. *See, e.g.,* Muldermans et al., 2001, J. Biotechnol. 74:277-302; Desmyter et al., 2001, J. Biol. Chem. 276:26285-90; Streltsov et al. (2005), Protein Science 14: 2901-2909. An antibody can be **"monospecific"** (that is, binding to only one kind of antigen), **"bispecific"** (that is, binding to two different antigens), or **"multispecific"** (that is, binding to more than one different antigen). Further, an antibody can be monovalent, bivalent, or multivalent, meaning that it can bind to one, two, or multiple antigen molecules at once, respectively. An antibody binds **"monovalently"** to a particular protein when one molecule of the antibody binds to only one molecule of the protein, even though the antibody may also bind to a different protein as well. That is, an antibody binds "monovalently," as meant herein, to two different proteins when it binds to only one molecule of each protein. Such an antibody is "bispecific" and binds to each of two different proteins "monovalently." An antibody can be **"monomeric,"** *i.e.,* comprising a single polypeptide chain. An antibody can comprise multiple polypeptide chains (**"multimeric"**) or can comprise two (**"dimeric"**), three (**"trimeric"**), or four (**"tetrameric"**) polypeptide chains. If multimeric, an antibody can be a homomultimer, *i.e.,* containing more than one molecule of only one kind of polypeptide chain, including homodimers, homotrimer, or homotetramers. Alternatively, a multimeric antibody can be a heteromultimer, *i.e.,* containing more than one different kind of polypeptide chain, including heterodimers, heterotrimers, or heterotetramers. An antibody can have a variety of possible formats including, for example, monospecific monovalent antibodies (as described in International Application WO 2009/089004 and US Publication 2007/0105199) that may inhibit or activate the molecule to which they bind, bivalent monospecific or bispecific dimeric Fv-Fc, scFv-Fc, or diabody Fc, monospecific monovalent scFv-Fc/Fc's, the multispecific binding proteins and dual variable domain immunoglobulins described in US Publication 2009/0311253 the V-C-Fc-V-C antibodies described herein, and the many formats for bispecific antibodies described in Chapters 1, 4, 5, 6, 7, 8, 9, 10, 11, 12, 13, 14 of BISPECIFIC ANTIBODIES, Kontermann, ed., Springer, 2011, among many other possible antibody formats.

A **"cancer cell antigen,"** as meant herein, is a protein expressed on the surface of a cancer cell. Some cancer cell antigens are also expressed on some normal cells, and some are specific to cancer cells. Cancer cell antigens can be highly expressed on the surface of a cancer cell. There are a wide variety of cancer cell antigens. Examples of cancer cell antigens include, without limitation, the following human proteins: epidermal growth factor receptor (EGFR), EGFRvIII (a mutant form of EGFR), melanoma-associated chondroitin sulfate proteoglycan (MCSP), mesothelin (MSLN), folate receptor 1 (FOLR1), and human epidermal growth factor 2 (HER2), among many others.

**"Chemotherapy,"** as used herein, means the treatment of a cancer patient with a **"chemotherapeutic agent"** that has cytotoxic or cytostatic effects on cancer cells. A **"chemotherapeutic agent"** specifically targets cells engaged in cell division and not cells that are not engaged in cell division. Chemotherapeutic agents directly interfere with processes that are intimately tied to cell division such as, for example, DNA replication, RNA synthesis, protein synthesis, the assembly, disassembly, or function of the mitotic spindle, and/or the synthesis or stability of molecules that play a role in these processes, such as nucleotides or amino acids. A chemotherapeutic agent therefore has cytotoxic or cytostatic effects on both cancer cells and other cells that are engaged in cell division. Chemotherapeutic agents are well-known in the art and include, for example: alkylating agents (*e.g.* busulfan, temozolomide, cyclophosphamide, lomustine (CCNU), methyllomustine, streptozotocin, *cis*-diamminedi-chloroplatinum, aziridinylbenzo-quinone, and thiotepa); inorganic ions (*e.g.* cisplatin and carboplatin); nitrogen mustards (*e.g.* melphalan hydrochloride, ifosfamide, chlorambucil, and mechlorethamine HCl); nitrosoureas (*e.g.* carmustine (BCNU)); anti-neoplastic antibiotics (*e.g.* adriamycin (doxorubicin), daunomycin, mitomycin C, daunorubicin, idarubicin, mithramycin, and bleomycin); plant derivatives (*e.g.* vincristine, vinblastine, vinorelbine, paclitaxel, docetaxel, vindesine, VP-16, and VM-26); antimetabolites (*e.g.* methotrexate with or without leucovorin, 5-fluorouracil with or without leucovorin, 5-fluorodeoxyuridine, 6-mercaptopurine, 6-thioguanine, cytarabine, 5-azacytidine, hydroxyurea, deoxycoformycin, gemcitabine, and fludarabine); podophyllotoxins (*e.g.* etoposide, irinotecan, and topotecan); as well as actinomycin D, dacarbazine (DTIC), mAMSA, procarbazine, hexamethylmelamine, pentamethylmelamine, L-asparaginase, and mitoxantrone, among many known in the art. *See e.g.* Cancer: Principles and Practice of Oncology, 4th Edition, DeVita et al., eds., J.B. Lippincott Co., Philadelphia, PA (1993). Alkylating agents and nitrogen mustard act by alkylating DNA, which restricts uncoiling and replication of strands. Methotrexate, cytarabine, 6-mercaptopurine, 5-fluorouracil, and gemcitabine interfere with nucleotide synthesis. Plant derivatives such a paclitaxel and vinblastine are mitotic spindle poisons. The podophyllotoxins inhibit topoisomerases, thus interfering with DNA replication. Antibiotics doxorubicin, bleomycin, and mitomycin interfere with DNA synthesis by intercalating between the bases of DNA (inhibiting uncoiling), causing strand breakage, and alkylating DNA, respectively. Other mechanisms of action include carbamoylation of amino acids (lomustine, carmustine), and depletion of asparagine pools (asparaginase). Merck Manual of Diagnosis and Therapy, 17th Edition, Section 11, Hematology and Oncology, 144. Principles of Cancer Therapy, Table 144-2 (1999). Specifically included among chemotherapeutic agents are those that directly affect the same cellular processes that are directly affected by the chemotherapeutic agents listed above.

A **"cleavage site for a protease,"** as meant herein, is an amino acid sequence that can be cleaved by a protease, such as, for example, a matrix metalloproteinase or a furin. Examples of such sites include Gly-Pro-Leu-Gly-Ile-Ala-Gly-Gln (SEQ ID NO:51) or Ala-Val-Arg-Trp-Leu-Leu-Thr-Ala (SEQ ID NO:59), which can cleaved by metalloproteinases, and Arg-Arg-Arg-Arg-Arg-Arg (SEQ ID NO:52), which is cleaved by a furin. Linkers including such cleavage site include, without limitation, linkers comprising the amino acid sequences of any one of SEQ ID NOs: 51-59. Further protease cleavage sites include, without limitation, the amino acid sequences of SEQ ID NOs:71-101. In therapeutic applications, the protease cleavage site can be cleaved by a protease that is produced by target cells, for example cancer cells or infected cells, or pathogens.

A drug or treatment is **"concurrently"** administered with a V-C-Fc-V-C antibody, as meant herein, if it is administered in the same general time frame as the antibody, optionally, on an ongoing basis. For example, if a patient is taking Drug A once a week on an ongoing basis and the V-C-Fc-V-C once every six months on an ongoing basis, Drug A and the V-C-Fc-V-C are concurrently administered, whether or not they are ever administered on the same day. Similarly, if the V-C-Fc-V-C is taken once per week on an ongoing basis and Drug A is administered only once or a few times on a daily basis, Drug A and the V-C-Fc-V-C are concurrently administered as meant herein. Similarly, if both Drug A and the V-C-Fc-V-C are administered for short periods of time either once or multiple times within a one month period, they are administered concurrently as meant herein as long as both drugs are administered within the same month. The V-C-Fc-V-C antibodies described herein can be administered before, after, or concurrently with a chemotherapeutic agent, a non-chemotherapeutic anti-neoplastic agent, radiation, or any other cancer treatment.

A **"conservative amino acid substitution,"** as meant herein, is a substitution of an amino acid with another amino acid with similar properties. Properties considered include chemical properties such as charge and hydrophobicity. Table 1 below lists substitutions for each amino acid that are considered to be conservative substitutions as meant herein.

**Table 1: Conservative Amino Acid Substitutions**

| Original Residue | Conservative Substitutions |
|---|---|
| Ala | Val, Leu, Ile |
| Arg | Lys, Gln, Asn |
| Asn | Gln |
| Asp | Glu |
| Cys | Ser, Ala |
| Gln | Asn |
| Glu | Asp |
| Gly | Pro, Ala |
| His | Asn, Gln, Lys, Arg |
| Ile | Leu, Val, Met, Ala, Phe, Norleucine |

(continued)

| Original Residue | Conservative Substitutions |
|---|---|
| Leu | Norleucine, Ile, Val, Met, Ala, Phe |
| Lys | Arg, Gln, Asn |
| Met | Leu, Phe, Ile |
| Phe | Leu, Val, Ile, Ala, Tyr |
| Pro | Ala |
| Ser | Thr, Ala, Cys |
| Thr | Ser |
| Trp | Tyr, Phe |
| Tyr | Trp, Phe, Thr, Ser |
| Val | Ile, Met, Leu, Phe, Ala, Norleucine |

A **"Fab fragment,"** as meant herein is a portion of a full length tetrameric antibody, for example an IgG antibody, resulting from cleavage of the antibody within or near the hinge region. A Fab fragment is at least a dimer containing a polypeptide chain comprising a VH and a CH1 region and another polypeptide comprising a VL and a CL region. In some embodiments, the polypeptide chains of a Fab fragment also comprise part or all of a hinge region. In some embodiments, the polypeptide chains of a Fab fragment can contain some or all of the cysteine residues in the hinge region. In such Fab fragments, two Fab fragments can be linked by one or more disulfide bonds to form tetrameric, bivalent Fab fragments known as F(ab)'₂ fragments. *See* Carayannopoulos and Capra, Immunoglobulins: Structure and Function, Ch. 9, pp. 283-314, *at* 284-285, *in* FUNDAMENTAL IMMUNOLOGY, THIRD EDITION, Paul, ed., Raven Press, New York, 1993. A **"half Fab fragment,"** as meant herein comprises a single polypeptide chain comprising a VH and a CH1 region, optionally plus part or all of a hinge region, or a VL and a CL region, optionally plus part or all of a hinge region.

As meant herein, an **"Fc region"** is a dimer consisting of two polypeptide chains joined by one or more disulfide bonds, each chain comprising part or all of a hinge domain plus a CH2 and a CH3 domain. Each of the polypeptide chains is referred to as an **"Fc polypeptide chain."** To distinguish the two Fc polypeptide chains, in some instances one is referred to herein as an **"A chain"** and the other is referred to as a **"B chain."** More specifically, the Fc regions contemplated for use with the present invention are IgG Fc regions, which can be mammalian, for example human, IgG1, IgG2, IgG3, or IgG4 Fc regions. Among human IgG1 Fc regions, at least two allelic types are known. In other embodiments, the amino acid sequences of the two Fc polypeptide chains can vary from those of a mammalian Fc polypeptide by no more than 15 substitutions, insertions, and/or deletions of a single amino acid per 100 amino acids of sequence relative to a mammalian Fc polypeptide amino acid sequence. In some embodiments, such variations can be "heterodimerizing alterations" that facilitate the formation of heterodimers over homodimers, an Fc alteration that extends half life, an alteration that reduces Fc gamma receptor (FcγR) binding, and/or an alteration that enhances ADCC.

An **"Fc alteration that extends half life,"** as meant herein is an alteration within an Fc polypeptide chain that lengthens the *in vivo* half life of a protein that contains the altered Fc polypeptide chain as compared to the half life of a similar protein containing the same Fc polypeptide, except that it does not contain the alteration. Such alterations can be included in an Fc polypeptide chain that is part of a V-C-Fc-V-C antibody as described herein. The alterations M252Y, S254T, and T256E (methionine at position 252 changed to tyrosine; serine at position 254 changed to threonine; and threonine at position 256 changed to glutamic acid; numbering according to EU numbering as shown in Table 2) are Fc alterations that extend half life and can be used together, separately or in any combination. These alterations and a number of others are described in detail in U.S. Patent 7,083,784. Similarly, M428L and N434S are Fc alterations that extend half life and can be used together, separately or in any combination. These alterations and a number of others are described in detail in U.S. Patent Application Publication 2010/0234575 and U.S. Patent 7,670,600. In addition, any substitution at one or more of the following sites can be considered an Fc alteration that extends half life as meant herein: 250, 251, 252, 259, 307, 308, 332, 378, 380, 428, 430, 434, and 436. Each of these alterations or combinations of these alterations can be used to extend the half life of a V-C-Fc-V-C antibody as described herein. Other alterations that can be used to extend half life are described in detail in International Application Publication WO 2013/096221. Some specific embodiments described in this application include insertions between positions 384 and 385 (EU numbering as shown in Table 2) that extend half life, including the following amino acid sequences: GGCVFNMFNCGG (SEQ ID NO:13), GGCHLPFAVCGG (SEQ ID NO:14), GGCGHEYMWCGG (SEQ ID NO:15), GGCWPLQDYCGG(SEQ ID NO:16), GGCMQMNKWCGG (SEQ ID NO:17), GGCDGRTKYCGG (SEQ ID NO:18), GGCALYPTNCGG (SEQ ID NO:19), GGCGKHWHQCGG (SEQ ID NO:20), GGCHSFKHFCGG (SEQ ID NO:21), GGCQGMWTWCGG (SEQ ID NO:22), GGCAQQWHHEYCGG (SEQ ID NO:23), and GGCERFHHACGG (SEQ ID NO:24), among others. V-C-Fc-V-C antibodies containing such insertions are contemplated.

A **"half life-extending moiety,"** as meant herein, is a molecule that extends the *in vivo* half life of a protein to which it is attached as compared to the *in vivo* half life of the protein without the half life-extending moiety. Methods for measuring half life are well known in the art. For example, a method for ascertaining half life is disclosed in Example 9. A half life-extending moiety can be a polypeptide, for example an Fc polypeptide chain or a polypeptide that can bind to albumin. The amino acid sequence of a domain of human fibronectin type III (Fn3) that has been engineered to bind to albumin is provided in SEQ ID NO:25, and various human IgG Fc polypeptide sequences are given in SEQ ID NOs:26-29. In alternate embodiments, a half life-extending moiety can be a non-polypeptide molecule. For example, a polyethylene glycol (PEG) molecule can be a half life-extending moiety.

**"Heterodimerizing alterations"** generally refer to alterations in the A and B chains of an Fc region that facilitate the formation of heterodimeric Fc regions, that is, Fc regions in which the A chain and the B chain of the Fc region do not have identical amino acid sequences. Such alterations can be included in an Fc polypeptide chain that is part of a V-C-Fc-V-C antibody as described herein. Heterodimerizing alterations can be asymmetric, that is, an A chain having a certain alteration can pair with a B chain having a different alteration. These alterations can facilitate heterodimerization and disfavor homodimerization. Whether hetero- or homodimers have formed can be assessed by size differences as determined by polyacrylamide gel electrophoresis in some situations or by other appropriate means such as differing charges or biophysical characteristics, including binding by antibodies or other molecules that recognize certain portions of the heterodimer including molecular tags. One example of such paired heterodimerizing alterations are the so-called "knobs and holes" substitutions. *See, e.g.,* US Patent 7,695,936 and US Patent Application Publication 2003/0078385. As meant herein, an Fc region that contains one pair of knobs and holes substitutions, contains one substitution in the A chain and another in the B chain. For example, the following knobs and holes substitutions in the A and B chains of an IgG1 Fc region have been found to increase heterodimer formation as compared with that found with unmodified A and B chains: 1) Y407T in one chain and T366Y in the other; 2) Y407A in one chain and T366W in the other; 3) F405A in one chain and T394W in the other; 4) F405W in one chain and T394S in the other; 5) Y407T in one chain and T366Y in the other; 6) T366Y and F405A in one chain and T394W and Y407T in the other; 7) T366W and F405W in one chain and T394S and Y407A in the other; 8) F405W and Y407A in one chain and T366W and T394S in the other; and 9) T366W in one polypeptide of the Fc and T366S, L368A, and Y407V in the other. This way of notating mutations can be explained as follows. The amino acid (using the one letter code) normally present at a given position in the CH3 region using the EU numbering system (which is presented in Edelman et al. (1969), Proc. Natl. Acad. Sci. 63: 78-85; *see also* Table 2 below) is followed by the EU position, which is followed by the alternate amino acid that is present at that position. For example, Y407T means that the tyrosine normally present at EU position 407 is replaced by a threonine. Alternatively or in addition to such alterations, substitutions creating new disulfide bridges can facilitate heterodimer formation. *See, e.g.,* US Patent Application Publication 2003/0078385. Such alterations in an IgG1 Fc region include, for example, the following substitutions: Y349C in one Fc polypeptide chain and S354C in the other; Y349C in one Fc polypeptide chain and E356C in the other; Y349C in one Fc polypeptide chain and E357C in the other; L351C in one Fc polypeptide chain and S354C in the other; T394C in one Fc polypeptide chain and E397C in the other; or D399C in one Fc polypeptide chain and K392C in the other. Similarly, substitutions changing the charge of a one or more residue, for example, in the $C_H3$-$C_H3$ interface, can enhance heterodimer formation as explained in US Patent Application Publication 2010/0286374. Such substitutions are referred to herein as **"charge pair substitutions,"** and an Fc region containing one pair of charge pair substitutions contains one substitution in the A chain and a different substitution in the B chain. General examples of charge pair substitutions include the following: 1) K409D or K409E in one chain plus D399K or D399R in the other; 2) K392D or K392E in one chain plus D399K or D399R in the other; 3) K439D or K439E in one chain plus E356K or E356R in the other; and 4) K370D or K370E in one chain plus E357K or E357R in the other. In addition, the substitutions R355D, R355E, K360D, or K360R in both chains can stabilize heterodimers when used with other heterodimerizing alterations. Specific charge pair substitutions can be used either alone or with other charge pair substitutions. Specific examples of single pairs of charge pair substitutions and combinations thereof include the following: 1) K409E in one chain plus D399K in the other; 2) K409E in one chain plus D399R in the other; 3) K409D in one chain plus D399K in the other; 4) K409D in one chain plus D399R in the other; 5) K392E in one chain plus D399R in the other; 6) K392E in one chain plus D399K in the other; 7) K392D in one chain plus D399R in the other; 8) K392D in one chain plus D399K in the other; 9) K409D and K360D in one chain plus D399K and E356K in the other; 10) K409D and K370D in one chain plus D399K and E357K in the other; 11) K409D and K392D in one chain plus D399K, E356K, and E357K in the other; 12) K409D and K392D on one chain and D399K on the other; 13) K409D and K392D on one chain plus D399K and E356K on the other; 14) K409D and K392D on one chain plus D399K and D357K on the other; 15) K409D and K370D on one chain plus D399K and D357K on the other; 16) D399K on one chain plus K409D and K360D on the other; and 17) K409D and K439D on one chain plus D399K and E356K on the other. Any of these heterodimerizing alterations can be used in the Fc regions of the V-C-Fc-V-C antibodies described herein.

An **"alteration that inhibits FcγR binding,"** as meant herein, is one or more insertions, deletions, or substitutions within an Fc polypeptide chain that inhibits the binding of FcγRIIA, FcγRIIB, and/or FcγRIIIA as measured, for example, by an ALPHALISA®-based competition binding assay (PerkinElmer, Waltham, MA). Such alterations can be included in an Fc polypeptide chain that is part of a V-C-Fc-V-C antibody, as described herein. More specifically, alterations that inhibit Fc gamma receptor (FcγR) binding include L234A, L235A, or any alteration that inhibits glycosylation at N297, including any substitution at N297. In addition, along with alterations that inhibit glycosylation at N297, additional alterations that stabilize a dimeric Fc region by creating additional disulfide bridges are also contemplated. Further examples of alterations that inhibit FcγR binding include a D265A alteration in one Fc polypeptide chain and an A327Q alteration in the other Fc polypeptide chain. Any such alterations can be used in the Fc regions of the V-C-Fc-V-C antibodies described herein.

An **"alteration that enhances ADCC,"** as meant herein is one or more insertions, deletions, or substitutions within an Fc polypeptide chain that enhances antibody dependent cell-mediated cytotoxicity (ADCC). Such alterations can be included in an Fc polypeptide chain that is part of a V-C-Fc-V-C antibody as described herein. Many such alterations are described in International Patent Application Publication WO 2012/125850. Such alterations can be included in an Fc polypeptide chain that is part of a V-C-Fc-V-C antibody as described herein. ADCC assays can be performed as follows. Cell lines that express high and lower amounts of a cancer cell antigen on the cell surface can be used as target cells. These target cells can be labeled with carboxyfluorescein succinimidyl ester (CFSE) and then washed once with phosphate buffered saline (PBS) before being deposited into 96-well microtiter plates with V-shaped wells. Purified immune effector cells, for example T cells or NK cells, can be added to each well. A monospecific antibody that binds to the cancer antigen and contains the alteration(s) being tested and an isotype-matched control antibody can be diluted in a 1:3 series and added to the wells. The cells can be incubated at 37°C with 5% $CO_2$ for 3.5 hrs. The cells can be spun down and re-suspended in 1x FACS buffer (1x phosphate buffered saline (PBS) containing 0.5% fetal bovine serum (FBS)) with the dye TO-PRO®-3 iodide (Molecular Probes, Inc. Corporation, Oregon, USA), which stains dead cells, before analysis by fluorescence activated cell sorting (FACS). The percentage of cell killing can be calculated using the following formula:

*(percent tumor cell lysis with bispecific – percent tumor cell lysis without bispecific)/*

*(percent total cell lysis – percent tumor cell lysis without bispecific)*

Total cell lysis is determined by lysing samples containing effector cells and labeled target cells without a bispecific molecule with cold 80% methanol. Exemplary alterations that enhance ADCC include the following alterations in the A and B chains of anFc region: (a) the A chain comprises Q311M and K334V substitutions and the B chain comprises L234Y, E294L, and Y296W substitutions or vice versa; (b) the A chain comprises E233L, Q311M, and K334V substitutions and the B chain comprises L234Y, E294L, and Y296W substitutions or vice versa; (c) the A chain comprises L234I, Q311M, and K334V substitutions and the B chain comprises L234Y, E294L, and Y296W substitutions or vice versa; (d) the A chain comprises S298T and K334V substitutions and the B chain comprises L234Y, K290Y, and Y296W substitutions or vice versa; (e) the A chain comprises A330M and K334V substitutions and the B chain comprises L234Y, K290Y, and Y296W substitutions or vice versa; (f) the A chain comprises A330F and K334V substitutions and the B chain comprises L234Y, K290Y, and Y296W substitutions or vice versa; (g) the A chain comprises Q311M, A330M, and K334V substitutions and the B chain comprises L234Y, E294L, and Y296W substitutions or vice versa; (h) the A chain comprises Q311M, A330F, and K334V substitutions and the B chain comprises L234Y, E294L, and Y296W substitutions or vice versa; (i) the A chain comprises S298T, A330M, and K334V substitutions and the B chain comprises L234Y, K290Y, and Y296W substitutions or vice versa; (j) the A chain comprises S298T, A330F, and K334V substitutions and the B chain comprises L234Y, K290Y, and Y296W substitutions or vice versa; (k) the A chain comprises S239D, A330M, and K334V substitutions and the B chain comprises L234Y, K290Y, and Y296W substitutions or vice versa; (I) the A chain comprises S239D, S298T, and K334V substitutions and the B chain comprises L234Y, K290Y, and Y296W substitutions or vice versa; (m) the A chain comprises a K334V substitution and the B chain comprises Y296W and S298C substitutions or vice versa; (n) the A chain comprises a K334V substitution and the B chain comprises L234Y, Y296W, and S298C substitutions or vice versa; (o) the A chain comprises L235S, S239D, and K334V substitutions and the B chain comprises L234Y, K290Y, and Y296W, substitutions or vice versa; (p) the A chain comprises L235S, S239D, and K334V substitutions and the B chain comprises L234Y, Y296W, and S298C substitutions or vice versa; (q) the A chain comprises Q311M and K334V substitutions and the B chain comprises L234Y, F243V, and Y296W substitutions or vice versa; (r) the A chain comprises Q311M and K334V substitutions and the B chain comprises L234Y, K296W, and S298C substitutions or vice versa; (s) the A chain comprises S239D and K334V substitutions and the B chain comprises L234Y, K290Y, and Y296W substitutions or vice versa; (t) the A chain comprises S239D and K334V substitutions and the B chain comprises L234Y, Y296W, and S298C substitutions or vice versa; (u) the A chain comprises F243V and K334V substitutions and the B chain comprises L234Y, K290Y, and Y296W, substitutions or vice versa; (v) the A chain comprises F243V and K334V substitutions and the B chain comprises L234Y, Y296W, and S298C substitutions or vice versa; (w) the A chain comprises E294L and K334V substitutions and the B chain comprises L234Y, K290Y, and Y296W substitutions or vice versa; (x) the A chain comprises E294L and K334V substitutions and the B chain comprises L234Y, Y296W, and S298C substitutions or vice versa; (y) the A chain comprises A330M and K334V substitutions and the B chain comprises L234Y and Y296W substitutions or vice versa; or (z) the A chain comprises A330M and K334V substitutions and the B chain comprises K290Y and Y296W substitutions or vice versa. Any such alterations can be used in the Fc regions of the V-C-Fc-V-C antibodies described herein.

A **"C-1, C-2, C-3, or C-4,"** as meant in the context of a V-C-Fc-V-C, is an immunoglobulin constant region, which follows an immunoglobulin variable region. C-1 follows a variable region at the amino terminal end of the first polypeptide chain of a V-C-Fc-V-C, and C-2 is at the carboxy terminal end. C-3 follows a variable region at the amino terminal end of the second polypeptide chain of a V-C-Fc-V-C, and C-4 is at the carboxy terminal end. C-1, C-2, C-3, and C-4 can have the same or different amino acid sequences, and they are either CL or CH1 regions.

A **"human"** protein or region of a protein, such as an immunoglobulin region or domain, has essentially the same amino acid sequence as the human protein or region thereof found in nature, with the proviso that the "human" protein sequence may contain some minor sequence variations relative to a human sequence found in nature. Such minor variations, not to exceed 15, 14, 13, 12, 11, 10, 9, 8, 7, 6, 5, 4, 3, 2, or 1 substitution(s), insertion(s), or deletion(s) of a single amino acid per 100 amino acids of sequence (taking into consideration the entire length of the protein or region), can include, for example, charge pair substitutions, as described herein, or one or more alterations that enhance ADCC, also as described herein, among other possible minor variations.

An **"IgG antibody,"** as meant herein, is an antibody consisting essentially of two immunoglobulin IgG heavy chains and two immunoglobulin light chains, which can be kappa or lambda light chains. More specifically, the heavy chains contain a VH, a CH1, a hinge region, a CH2, and a CH3, while the light chains contain a VL and a CL. Numerous sequences of such immunoglobulin regions are known in the art. *See, e.g.,* Kabat et al. in SEQUENCES OF IMMUNOLOGICAL INTEREST, Public Health Service N.I.H., Bethesda, MD, 1991.

An **"immune effector cell,"** as meant herein, is a cell that is involved in the mediation of a cytolytic immune response, including, for example, T cells, NK cells, macrophages, or neutrophils. The V-C-Fc-V-C antibodies described herein bind to an antigen that is part of a molecule, optionally a protein, expressed on the surface of an immune effector cell. Such molecules are referred to herein as **"effector cell molecules"** or, in the case of proteins, **"effector cell proteins."**

An **"immunoglobulin heavy chain,"** as meant herein, consists essentially of a VH region, a CH1 region, a hinge region, a CH2 region, a CH3 region in that order, and, optionally, a region downstream of the CH3 region in some isotypes. Close variants of an immunoglobulin heavy chain containing no more than 15 amino acid substitutions, insertions, and/or deletions of a single amino acid per 100 amino acids relative to a known or naturally occurring immunoglobulin heavy chain amino acid sequence are encompassed within what is meant by an immunoglobulin heavy chain.

A **"immunoglobulin light chain,"** as meant herein, consists essentially of a VL and a CL. Close variants of an immunoglobulin light chain containing no more than 15 amino acid substitutions, insertions, and/or deletions of a single amino acid per 100 amino acids relative to a known or naturally occurring immunoglobulin light chain amino acid sequence are encompassed within what is meant by an immunoglobulin light chain.

An **"immunoglobulin variable region,"** as meant herein, is a VH region, a VL region, or a variant thereof. Close variants of an immunoglobulin variable region containing no more than 15 amino acid substitutions, insertions, and/or deletions of a single amino acid per 100 amino acids relative to a known or naturally occurring immunoglobulin variable region amino acid sequence are encompassed within what is meant by an immunoglobulin variable region. Many examples of VH and VL regions are known in the art, such as, for example, those disclosed by Kabat et al. in SEQUENCES OF IMMUNOLOGICAL INTEREST, Public Health Service N.I.H., Bethesda, MD, 1991. Based on the extensive sequence commonalities in the less variable portions of the VH and VL regions, the position within a sequence of more variable regions, and the predicted tertiary structure, one of skill in the art can recognize an immunoglobulin variable region by its sequence. *See, e.g.,* Honegger and Plückthun (2001), J. Mol. Biol. 309: 657-670.

An immunoglobulin variable region contains three hypervariable regions, known as complementarity determining region 1 (CDR1), complementarity determining region 2 (CDR2), and complementarity determining region 3 (CDR3). These regions form the antigen binding site of an antibody. The CDRs are embedded within the less variable framework regions (FR1-FR4). The order of these subregions within an immunoglobulin variable region is as follows: FR1-CDR1-FR2-CDR2-FR3-CDR3-FR4. Numerous sequences of immunoglobulin variable regions are known in the art. *See, e.g.,* Kabat et al., SEQUENCES OF PROTEINS OF IMMUNOLOGICAL INTEREST, Public Health Service N.I.H., Bethesda, MD, 1991.

CDRs can be located in a VH region sequence in the following way. CDR1 starts at approximately residue 31 of the mature VH region and is usually about 5-7 amino acids long, and it is almost always preceded by a Cys-Xxx-Xxx-Xxx-Xxx-Xxx-Xxx-Xxx-Xxx (SEQ ID NO:30) (where "Xxx" is any amino acid). The residue following the heavy chain CDR1 is almost always a tryptophan, often a Trp-Val, a Trp-Ile, or a Trp-Ala. Fourteen amino acids are almost always between the last residue in CDR1 and the first in CDR2, and CDR2 typically contains 16 to 19 amino acids. CDR2 may be immediately preceded by Leu-Glu-Trp-Ile-Gly (SEQ ID NO:31) and may be immediately followed by Lys/Arg-Leu/Ile/Val/Phe/Thr/Ala-Thr/Ser/Ile/Ala. Other amino acids may precede or follow CDR2. Thirty two amino acids are almost always between the last residue in CDR2 and the first in CDR3, and CDR3 can be from about 3 to 25 residues long. A Cys-Xxx-Xxx almost always immediately precedes CDR3, and a Trp-Gly-Xxx-Gly (SEQ ID NO: 32) almost always follows CDR3.

Light chain CDRs can be located in a VL region in the following way. CDR1 starts at approximately residue 24 of the mature antibody and is usually about 10 to 17 residues long. It is almost always preceded by a Cys. There are almost always 15 amino acids between the last residue of CDR1 and the first residue of CDR2, and CDR2 is almost always 7 residues long. CDR2 is typically preceded by Ile-Tyr, Val-Tyr, Ile-Lys, or Ile-Phe. There are almost always 32 residues between CDR2 and CDR3, and CDR3 is usually about 7 to 10 amino acids long. CDR3 is almost always preceded by Cys and usually followed by Phe-Gly-Xxx-Gly (SEQ ID NO:33).

A **"linker,"** as meant herein, is a peptide that links two polypeptides, which can be two immunoglobulin variable regions in the context of a V-C-Fc-V-C antibody. A linker can be from 2-40 amino acids in length. In some embodiments, a linker can be about 2-35, 2-30, or 3-25 amino acids long. In some embodiments, a linker can be a peptide no more than about 35, 30, 25, 14, 13, 12, 11, 10, 9, 8, 7, 6, or 5 amino acids long. In other embodiments, a linker can be about 5-35, 5-30, 4-20, 10-20, or 20-35 amino acids long. In other embodiments, a linker can be at least about, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids long and/or no more than about 40, 35, 30, 25, 20, or 15 amino acids long. Exemplary linkers include, for example, the amino acid sequences TVAAP (SEQ ID NO:34), ASTKGP (SEQ ID NO:35),GGGGSGGGGS (SEQ ID NO:36), GGGGSAAA (SEQ ID NO:37), GGGGSGGGGSGGGGS (SEQ ID NO:38), (GGGGS)$_n$ (SEQ ID NO:50; where n is a number from 1 to 10), and AAA, among many others. In some embodiments linkers in different parts of the V-C-Fc-V-C molecule can have different sequences and/or different lengths. Different lengths of the linkers between the CH3 regions and the variable regions that follow them might allow these variable regions to be more accessible to allow better antigen binding. In some embodiments the linker between the CH3 region and the second segment containing a variable region plus a CL or CH1 region on one polypeptide chain of a V-C-Fc-V-C can contain an amino acid sequence that can be cleaved by a protease, for example, a furin protease, which can be present in a host cell, a metalloproteinase, for example, matrix metalloproteinase 2 (MMP2) or MMP9, a membrane anchored serine protease, which can be present of the surface of a target cell, or any other appropriate protease. Examples of such linkers include, without limitation, the following sequences: GGGGSRRRRRRGGGGS (SEQ ID NO:53), GGGGSGPLGIAGQGGGGS (SEQ ID NO:54), GGGGSGGGGSGLEVLFQGPSGGGGSGGGGS (SEQ ID NO:55), GGGGSGGGGSGQSSRHRRALGGGGSGGGGS (SEQ ID NO:56), GGGGSGGGGSGGPLGMLSQSGGGGSGGGGS (SEQ ID NO:57), GGGGSGGGGSGGGGRRGGSGGGGSGGGGS (SEQ ID NO:58), and AVRWLLTA (SEQ ID NO:59).

A V-C-Fc-V-C antibody **"mediates cytolysis of a target cell by an immune effector cell,"** as meant herein, when addition of an amount from 0.001 pM to 20000 pM of the V-C-Fc-V-C antibody to a cell cytolysis assay as described herein effectively elicits cytolysis of the target cells.

**"Non-chemotherapeutic anti-neoplastic agents"** are chemical agents, compounds, or molecules having cytotoxic or cytostatic effects on cancer cells other than chemotherapeutic agents. Non-chemotherapeutic antineoplastic agents may, however, be targeted to interact directly with molecules that indirectly affect cell division such as cell surface receptors, including receptors for hormones or growth factors. However, non-chemotherapeutic antineoplastic agents do not interfere directly with processes that are intimately linked to cell division such as, for example, DNA replication, RNA synthesis, protein synthesis, or mitotic spindle function, assembly, or disassembly. Examples of non-chemotherapeutic anti-neoplastic agents include inhibitors of Bcl2, inhibitors of farnesyltransferase, anti-estrogenic agents such as tamoxifen, anti-androgenic compounds, interferon, arsenic, retinoic acid, retinoic acid derivatives, antibodies targeted to tumor-specific antigens, and inhibitors of the Bcr-Abl tyrosine kinase (*e.g.,* the small molecule STI-571 marketed under the trade name GLEEVEC™ by Novartis, New York and New Jersey, USA and Basel, Switzerland), among many possible non-chemotherapeutic anti-neoplastic agents.

A **"signal sequence,"** is a hydrophobic amino acid sequence that mediates insertion of a secreted or transmembrane protein through the membrane bounding the endoplasmic reticulum (ER) in a eukaryotic cell. "Signal sequences," as meant herein are amino-terminal hydrophobic sequences which are usually enzymatically removed following the insertion of part or all of the protein through the ER membrane into the lumen of the ER. Thus, it is known in the art that a signal sequence can be present as part of a precursor form of a secreted or transmembrane protein, but will generally be absent from the mature form of the protein. When a protein is said to comprise a signal sequence, it is to be understood that, although a precursor form of the protein does contain the signal sequence, a mature form of the protein will likely not contain the signal sequence. Signal sequences contain a residue adjacent to and immediately upstream from the cleavage site (position -1) and another residue at position -3, which are important for this enzymatic cleavage. Nielsen et al. (1997), Protein Eng. 10(1):1-6; von Heijne (1983), Eur. J. Biochem. 133:17-21; von Heijne (1985), J. Mol. Biol. 184:99-105. Signal sequences can be identified as described by Nielsen et al. (*supra*). Examples of signal peptides or sequences that are functional in mammalian host cells include the following: the signal sequence for interleukin-7 (IL-7) described in US Patent 4,965,195; the signal sequence for interleukin-2 receptor described in Cosman et al. ((1984), Nature 312:768); the interleukin-4 receptor signal peptide described in EP Patent 0 367 566; the type I interleukin-1 receptor signal sequence described in US Patent 4,968,607; the type II interleukin-1 receptor signal peptide described in EP Patent 0 460 846; the signal sequence of human IgK; and the signal sequence of human growth hormone. Many other signal sequences are known in the art. Many amino acid sequences described herein include signal sequences, for example, SEQ ID NOs:1, 3, 5, 7, 9, 11, 41, 44, 46, 48, 62, and 64.

A **"target cell"** is a cell that a V-C-Fc-V-C antibody, as described herein, binds to and that is involved in mediating a disease. In some cases, a target cell can be a cell that is ordinarily involved in mediating an immune response, but is also involved in the mediation of a disease. For example in B cell lymphoma, a B cell, which is ordinarily involved in mediating immune response, can be a target cell. In some embodiments, a target cell is a cancer cell, a cell infected with a pathogen, or a cell involved in mediating an autoimmune or inflammatory disease. The V-C-Fc-V-C antibody can bind to the target cell via binding to an antigen on a **"target cell molecule,"** which can be a **"target cell protein,"** which is a molecule that is displayed on the surface of the target cell, possibly a protein that may be highly expressed. Alternatively, a "target cell" of a V-C-Fc-V-C can be a pathogen, which can be, without limitation, a virus, a bacterium, a fungus, a protozoan, or a helminth.

**"Tumor burden"** refers to the number of viable cancer cells, the number of tumor sites, and/or the size of the tumor(s) in a patient suffering from a cancer. A reduction in tumor burden can be observed, for example, as a reduction in the amount of a tumor-associated antigen or protein in a patient's blood or urine, a reduction in the number of tumor cells or tumor sites, and/or a reduction in the size of one or more tumors.

A **"therapeutically effective amount"** of a V-C-Fc-V-C antibody as described herein is an amount that has the effect of, for example, reducing or eliminating the tumor burden of a cancer patient or reducing or eliminating the symptoms of any disease condition that the protein is used to treat. A therapeutically effective amount need not completely eliminate all symptoms of the condition, but may reduce severity of one or more symptoms or delay the onset of more serious symptoms or a more serious disease that can occur with some frequency following the treated condition.

**"Treatment"** of any disease mentioned herein encompasses an alleviation of at least one symptom of the disease, a reduction in the severity of the disease, or the delay or prevention of disease progression to more serious symptoms that may, in some cases, accompany the disease or lead to at least one other disease. Treatment need not mean that the disease is totally cured. A useful therapeutic agent needs only to reduce the severity of a disease, reduce the severity of one or more symptoms associated with the disease or its treatment, or delay the onset of more serious symptoms or a more serious disease that can occur with some frequency following the treated condition.

When it is said that a named VH/VL pair of immunoglobulin variable regions can bind to a target cell or an immune effector cell **"when they are part of an IgG antibody or scFv antibody,"** it is meant that an IgG antibody that contains the named VH region in both heavy chains and the named VL region in both light chains or the scFv that contains the VH/VL pair can bind to the target cell or the immune effector cell. A binding assay is described in Example 2. One of skill in the art could construct an IgG or scFv antibody containing the desired sequences given the knowledge in the art.

### *V-C-Fc-V-C Antibodies*

In the most general sense, a V-C-Fc-V-C antibody as described herein comprises two polypeptide chains having different amino acid sequences, which, together, can bind to two different antigens. Such a molecule can have a half life between a few hours and a few days or from a few days to one or more weeks. A diagram of a structure of a V-C-Fc-V-C antibody is shown in Figure 1. Optionally, linkers can occur between any of the regions diagrammed in Figure 1. The first polypeptide chain can comprise a first segment comprising a first variable region followed by a CH1 or CL region followed by an Fc polypeptide chain comprising part or all of a hinge region, a CH2 region, and a CH3 region, which can optionally be followed by a linker and then a second segment comprising a second variable region followed by a CH1 or CL region, which, optionally, can be followed by part or all of a hinge region. The second polypeptide chain of this embodiment can comprise a third segment comprising a third variable region followed by a CL or CH1 region followed by an Fc polypeptide chain comprising part or all of a hinge region, a CH2 region, and a CH3 region, which is followed by an optional linker and then a fourth segment comprising a fourth variable region followed by a CL or CH1 region, which, optionally, can be followed by part of all of a hinge region. If the first segment comprises a CH1 region, then the third segment can comprise a CL region. Similarly, if the first segment comprises a CL region, then the third segment can comprise a CH1 region. Also, if the second segment comprises a CH1 region, then the fourth segment can comprise a CL region. Similarly, if the second segment comprises a CL region, then the fourth segment can comprise a CH1 region. Also, if the first variable region is a VH region, then the third variable region can be a VL region, and if the first variable region is a VL region, then the third variable can be a VH region. Similarly, if the second variable region is a VH region, then the fourth variable region can be a VL region, and if the second variable region is a VL region, then the fourth variable region can be a VH region. Optionally, the VH/VL pairs containing the first and third variable regions and the second and fourth variable regions can each bind to a target cell, which may be a pathogen including a virus, or an immune effector cell when they are part of an IgG antibody or scFv antibody. In some embodiments, the V-C-Fc-V-C antibody can bind to an immune effector cell and a target cell.

In one embodiment shown in Figure 2, the first polypeptide chain (at left of panel A, B, C, or D in Figure 2) can comprise a first segment comprising a first VH-1 region followed by a CH1-1 region followed by an Fc polypeptide chain comprising part or all of a hinge region, a CH2-1 region, and a CH3-1 region, which is followed by a linker and then a second segment comprising a VH-2 region followed by a CH1-2 region and, optionally, part or all of a hinge region. The second polypeptide chain (at right of panel A, B, C, or D in Figure 2) of this embodiment can comprise a third segment comprising a VL-1 region and a CL-1 region followed by an Fc polypeptide chain comprising part or all of a hinge region, a CH2-2 region, and a CH3-2 region, which is followed by a linker and then a fourth segment comprising a second VL-2 region followed by a CL-2 region and, optionally, part or all of a hinge region. In these designations, the numbers following the dash, i.e., "-1" or "-2", indicate that the indicated domains are distinct domains that can have different amino acids sequence. For example VH-1 and VH-2 are both heavy chain variable regions, but they are in distinct positions within the V-C-Fc-V-C antibody and can have different amino acid sequences. Similar considerations apply for VL-1, VL-2, CH1-1, CH1-2, etc.

In another embodiment, the first polypeptide chain can comprise a first segment comprising a VH-1 region and a CH1-1 region followed by an Fc polypeptide chain comprising part or all of a hinge region, a CH2-1 region, and a CH3-1 region, which is followed by a linker and then a second segment comprising a VL-2 region followed by a CL-2 region and, optionally, part or all of a hinge region.. The second polypeptide chain of this embodiment can comprise a third segment comprising a VL-1 region and a CL-1 region followed by an Fc polypeptide chain comprising part or all of a hinge region, a CH2-2 region, and a CH3-2 region, which is followed by a linker and then a fourth segment comprising a VH-2 region followed by a CH1-2 region and, optionally, part or all of a hinge region.

In a further embodiment, the first polypeptide chain can comprise a first segment comprising a VL-1 region and a CH1-1 region followed by an Fc polypeptide chain comprising part or all of a hinge region, a CH2-1 region, and a CH3-1 region, which is followed by a linker and then a second segment comprising a VH-2 region followed by a CH1-2 region and, optionally, part or all of a hinge region.. The second polypeptide chain of this embodiment can comprise a third segment comprising a VH-1 region and a CL-1 region followed by an Fc polypeptide chain comprising part or all of a hinge region, a CH2-2 region, and a CH3-2 region, which is followed by a linker and then a fourth segment comprising a VL-2 region followed by a CL-2 region and, optionally, part or all of a hinge region.

In a further embodiment, the first polypeptide chain can comprise a first segment comprising a VL-1 region and a CH1-1 region followed by an Fc polypeptide chain comprising part or all of a hinge region, a CH2-1 region, and a CH3-1 region, which is followed by a linker and then a second segment comprising a VL-2 region followed by a CH1-2 region and, optionally, part or all of a hinge region.. The second polypeptide chain of this embodiment can comprise a third segment comprising a VH-1 region and a CL-1 region followed by an Fc polypeptide chain comprising part or all of a hinge region, a CH2-2 region, and a CH3-2 region, which is followed by a linker and then a fourth segment comprising a VH-2 region followed by a CL-2 region and, optionally, part or all of a hinge region.

In still another embodiment, the first polypeptide chain can comprise a first segment comprising a VH-1 region and a CH1-1 region followed by an Fc polypeptide chain comprising part or all of a hinge region, a CH2-1 region, and a CH3-1 region, which is followed by a linker and then a second segment comprising a VL-2 region followed by a CH1-2 region and, optionally, part or all of a hinge region.. The second polypeptide chain of this embodiment can comprise a third segment comprising a VL-1 region and a CL-1 region followed by an Fc polypeptide chain comprising part or all of a hinge region, a CH2-2 region, and a CH3-2 region, which is followed by a linker and then a fourth segment comprising a VH-2 region followed by a CL-2 region and, optionally, part or all of a hinge region.

In any of the embodiments described above, the identities of the first polypeptide chain and the second polypeptide chain can be switched.

The VH-1/VL-1 and VH-2/VL-2 pairs of immunoglobulin variable regions can each bind to a target cell, including a pathogen, or an immune effector cell when they are part of an IgG antibody or scFv antibody. The V-C-Fc-V-C antibody can bind to an immune effector cell and a target cell.

An optional linker between an Fc polypeptide chain and the second or fourth segment, *i.e.,* between the CH3-1 or CH3-2 region and the VH-2 or VL-2 region, can be present or absent and can have the same or a different amino acid sequence in the two polypeptide chains of a V-C-Fc-V-C antibody. These linkers can play a role in the structure and function of the antibody. If the linker is short, *i.e.,* less than 12 amino acids long, it will limit flexibility. If the linker is at least 15 amino acids long, it will allow much more flexibility. In some embodiments, these linkers on the two polypeptide chains of the V-C-Fc-V-C can have different lengths and/or sequences, which can make the VL-2/VH-2 more accessible for binding.

Further, optional linkers can occur between any of the domains in a V-C-Fc-V-C.

In addition, a linker downstream from an Fc polypeptide chain in one, but not both, polypeptide chains can comprise an amino acid sequence cleavable by a protease. In embodiments where such a linker is cleaved, the segment downstream from the Fc region can be more accessible and can exhibit higher affinity for the molecule it binds to than it would if the linker were not cleaved. This can correlate with higher activity in a binding and/or a cell killing assay as described in the Examples below. The linker can be cleavable by any protease, for example, a furin or a matrix metalloproteinase. If the protease is a furin, it may be present in a host cell used to produce the V-C-Fc-V-C, and the V-C-Fc-V-C may be secreted from the host cell in a cleaved form. Alternatively, protease digestion can occur after the V-C-Fc-V-C has been produced or purified and before it is administered to a patient. As a further alternative, a V-C-Fc-V-C can contain a protease site that is cleaved *in vivo* following administration to a patient, for example by a metalloproteinase secreted by a cancer cell. Such a V-C-Fc-V-C has the advantage of being fully active only in the presence of appropriate target cells. Exemplary sequences of linkers containing protease cleavage sites are provided in SEQ ID NOs: 51-59 and 71-101.

A V-C-Fc-V-C antibody as described herein can contain an additional, or alternative, half-life extending moiety besides the Fc region. This moiety could be, for example, be albumin, an albumin fragment, a moiety that binds to albumin or to the neonatal Fc receptor (FcRn), a derivative of fibronectin that has been engineered to bind albumin (*e.g.*, having the amino acid sequence of SEQ ID NO:25) or a fragment thereof, a peptide, a single domain protein fragment, or other polypeptide that can increase serum half life. In alternate embodiments, a half life-extending moiety can be a non-polypeptide molecule such as, for example, polyethylene glycol (PEG).

Sequences of human IgG1, IgG2, IgG3, and IgG4 Fc polypeptides that could be used in a V-C-Fc-V-C antibody are provided in SEQ ID NOs:26-29. Variants of these sequences containing one or more heterodimerizing alterations, one or more Fc alteration that extends half life, one or more alteration that enhances ADCC, and/or one or more alteration that inhibits Fc gamma receptor (FcγR) binding are also contemplated, as are other close variants containing not more than 15, 10, 8, 5, or 3 deletions, insertions, or substitutions of a single amino acid per 100 amino acids of sequence.

As explained above, an Fc polypeptide chain comprises all or part of a hinge region followed by a CH2 and a CH3 region. The Fc polypeptide chain can be of mammalian (for example, human, mouse, rat, rabbit, dromedary, or new or old world monkey), avian, or shark origin. In addition, as explained above, an Fc polypeptide chain can have a limited number alterations. For example, an Fc polypeptide chain can comprise one or more heterodimerizing alterations, one or more alteration(s) that inhibit(s) binding to FcγR, or one or more alteration(s) that increase(s) binding to FcRn. Exemplary amino acid sequences of pairs of polypeptide chains that make up a V-C-Fc-V-C antibody containing an Fc region include, without limitation, the following pairs of sequences: SEQ ID NOs: 1 and 3; SEQ ID NOs: 5 and 7; SEQ ID NOs: 9 and 11, SEQ ID NOs: 46 and 48, SEQ ID NOs: 11 and 62, and SEQ ID NOs: 11 and 64.

In some embodiments the amino acid sequences of the Fc polypeptide chains can be mammalian, for example human amino acid sequences. The isotype of the Fc polypeptide can be IgG, such as IgG1, IgG2, IgG3, or IgG4, IgA, IgD, IgE, or IgM. Table 2 below shows an alignment of the amino acid sequences of human IgG1, IgG2, IgG3, and IgG4 sequences.

## Table 2: Amino acid sequences of human IgG Fc polypeptide chains

```
IgG1        --------------------------------------------------
IgG2        --------------------------------------------------
IgG3        ELKTPLGDTTHTCPRCPEPKSCDTPPPCPRCPEPKSCDTPPPCPRCP
IgG4        --------------------------------------------------


            225       235       245       255       265       275
            *         *         *         *         *         *
IgG1    EPKSCDKTHTCPPCPAPELLGGPSVFLFPPKPKDTLMISRTPEVTCVVVDVSHEDPEVKF
IgG2    ERKCCVE---CPPCPAPPVA-GPSVFLFPPKPKDTLMISRTPEVTCVVVDVSHEDPEVQF
IgG3    EPKSCDTPPPCPRCPAPELLGGPSVFLFPPKPKDTLMISRTPEVTCVVVDVSHEDPEVQF
IgG4    ESKYG---PPCPSCPAPEFLGGPSVFLFPPKPKDTLMISRTPEVTCVVVDVSQEDPEVQF


            285       295       305       315       325       335
            *         *         *         *         *         *
IgG1    NWYVDGVEVHNAKTKPREEQYNSTYRVVSVLTVLHQDWLNGKEYKCKVSNKALPAPIEKT
IgG2    NWYVDGMEVHNAKTKPREEQFNSTFRVVSVLTVVHQDWLNGKEYKCKVSNKGLPAPIEKT
IgG3    KWYVDGVEVHNAKTKPREEQYNSTFRVVSVLTVLHQDWLNGKEYKCKVSNKALPAPIEKT
IgG4    NWYVDGVEVHNAKTKPREEQFNSTYRVVSVLTVLHQDWLNGKEYKCKVSNKGLPSSIEKT


            345       355       365       375       385       395
            *         *         *         *         *         *
IgG1    ISKAKGQPREPQVYTLPPSREEMTKNQVSLTCLVKGFYPSDIAVEWESNGQPENNYKTTP
IgG2    ISKTKGQPREPQVYTLPPSREEMTKNQVSLTCLVKGFYPSDIAVEWESNGQPENNYKTTP
IgG3    ISKTKGQPREPQVYTLPPSREEMTKNQVSLTCLVKGFYPSDIAVEWESSGQPENNYNTTP
IgG4    ISKAKGQPREPQVYTLPPSQEEMTKNQVSLTCLVKGFYPSDIAVEWESNGQPENNYKTTP


            405       415       425       435       445
            *         *         *         *         *
IgG1    PVLDSDGSFFLYSKLTVDKSRWQQGNVFSCSVMHEALHNHYTQKSLSLSPGK (SEQ ID NO:26)



IgG2    PMLDSDGSFFLYSKLTVDKSRWQQGNVFSCSVMHEALHNHYTQKSLSLSPGK (SEQ ID NO:27)
IgG3    PMLDSDGSFFLYSKLTVDKSRWQQGNIFSCSVMHEALHNRFTQKSLSLSPGK (SEQ ID NO:28)
IgG4    PVLDSDGSFFLYSRLTVDKSRWQEGNVFSCSVMHEALHNHYTQKSLSLSLGK (SEQ ID NO:29)
```

The numbering shown in Table 2 is according the EU system of numbering, which is based on the sequential numbering of the constant region of an IgG1 antibody. Edelman et al. (1969), Proc. Natl. Acad. Sci. 63: 78-85. Thus, it does not accommodate the additional length of the IgG3 hinge well. It is nonetheless used here to designate positions in an Fc polypeptide chain because it is still commonly used in the art to refer to positions in Fc polypeptide chains. The hinge regions of the IgG1, IgG2, and IgG4 Fc polypeptides extend from about position 216 to about 230. It is clear from the alignment that the IgG2 and IgG4 hinge regions are each three amino acids shorter than the IgG1 hinge. The IgG3 hinge is much longer, extending for an additional 47 amino acids upstream. The CH2 region extends from about position 231 to 340, and the CH3 region extends from about position 341 to 447.

Naturally occurring amino acid sequences of Fc polypeptide chains can be varied slightly. Such variations can include no more than 15, 10, 8, 5, or 3 insertions, deletions, or substitutions of a single amino acid per 100 amino acids of sequence of a naturally occurring Fc polypeptide chain. If there are substitutions, they can be conservative amino acid substitutions, as defined above. The Fc polypeptide chains within the first and second polypeptide chains of a V-C-Fc-V-C antibody can differ in amino acid sequence. In some embodiments, they can include "heterodimerizing alterations," for example, charge pair substitutions, as defined above, that facilitate heterodimer formation. Further, the Fc polypeptide chain portions of a V-C-Fc-V-C antibody can also contain alterations that inhibit Fc y R binding. Such mutations are described above and in Xu et al. (2000), Cell. Immunol. 200(1): 16-26. The Fc polypeptide chain portions can also include an "Fc alteration that extends half life," as described above, including those described in, e.g., US Patents 7,037,784, 7,670,600, and 7,371,827, US Patent Application Publication 2010/0234575, and International Application PCT/US2012/070146. Further, an Fc polypeptide can comprise "alterations that enhance ADCC," as defined above.

A V-C-Fc-V-C antibody as described herein can bind to an immune effector cell through an antigen that can be part of an effector cell molecule and can bind to a target cell through an antigen that can be part of a target cell molecule or protein. Some effector cell molecules, which can be effector cell proteins, are described below. Similarly, possible target cell molecules, which can be target cell proteins, are also described below. A V-C-Fc-V-C antibody can bind to any combination of an effector cell molecule and a target cell molecule, which can be engaged noncovalently by the V-C-Fc-V-C antibody. Alternatively, a V-C-Fc-V-C antibody may bind to an antigen displayed on target and/or effector cells that is not part of a protein.

### Nucleic Acids Encoding V-C-Fc-V-C Antibodies

Provided are nucleic acids encoding the V-C-Fc-V-C antibodies described herein. Numerous nucleic acid sequences encoding immunoglobulin regions including VH, VL, hinge, CH1, CH2, CH3, and CH4 regions are known in the art. *See, e.g.,* Kabat et al. in SEQUENCES OF IMMUNOLOGICAL INTEREST, Public Health Service N.I.H., Bethesda, MD, 1991. Using the guidance provided herein, one of skill in the art could combine such nucleic acid sequences and/or other nucleic acid sequences known in the art to create nucleic acid sequences encoding the V-C-Fc-V-C antibodies described herein. Exemplary pairs of nucleic acids encoding V-C-Fc-V-C antibodies include the following: SEQ ID NOs:2 and 4; SEQ ID NOs:6 and 8; SEQ ID NOs:10 and 12; SEQ ID NOs:47 and 49, SEQ ID NOs:12 and 63, and SEQ ID NOs:12 and 65.

In addition, nucleic acid sequences encoding V-C-Fc-V-C antibodies described herein can be determined by one of skill in the art based on the amino acid sequences provided herein and knowledge in the art. Numerous sequences of immunoglobulin regions are known in the art and can be amplified and joined together using polymerase chain reaction. Besides more traditional methods of producing cloned DNA segments encoding a particular amino acid sequence, companies such as DNA 2.0 (Menlo Park, CA, USA) and Blue Heron (Bothell, WA, USA), among others, now routinely produce chemically synthesized, gene-sized DNAs of any desired sequence to order, thus streamlining the process of producing such DNAs.

### Methods of Making V-C-Fc-V-C Antibodies

The V-C-Fc-V-C antibodies described herein can be made using methods well known in the art. For example, nucleic acids encoding the two polypeptide chains of a V-C-Fc-V-C antibody can be introduced into a cultured host cell by a variety of known methods, such as, for example, transformation, transfection, electroporation, bombardment with nucleic acid-coated microprojectiles, etc. In some embodiments, the nucleic acids encoding the V-C-Fc-V-C antibodies can be inserted into a vector appropriate for expression in the host cells before being introduced into the host cells. Typically such vectors can contain sequence elements enabling expression of the inserted nucleic acids at the RNA and protein levels. Such vectors are well known in the art, and many are commercially available. The host cells containing the nucleic acids can be cultured under conditions so as to enable the cells to express the nucleic acids, and the resulting V-C-Fc-V-C antibodies can be collected from the cell mass or the culture medium. Alternatively, the V-C-Fc-V-C antibodies can be produced in *vivo,* for example in plant leaves (*see, e.g.*, Scheller et al. (2001), Nature Biotechnol. 19: 573-577 and references cited therein), bird eggs (*see, e.g.,* Zhu et al. (2005), Nature Biotechnol. 23: 1159-1169 and references cited therein), or mammalian milk (*see, e.g.,* Laible et al. (2012), Reprod. Fertil. Dev. 25(1): 315).

A variety of cultured host cells can be used including, for example, bacterial cells such as *Escherichia coli or Bacilis steorothermophilus,* fungal cells such as *Saccharomyces cerevisiae* or *Pichia pastoris,* insect cells such as lepidopteran insect cells including *Spodoptera frugiperda* cells, or mammalian cells such as Chinese hamster ovary (CHO) cells, baby hamster kidney (BHK) cells, monkey kidney cells, HeLa cells, human hepatocellular carcinoma cells, or human embryonic kidney (HEK) 293 cells, among many others.

### Immune Effector Cells and Effector Cell Molecules

A V-C-Fc-V-C antibody as described herein can bind to a molecule expressed on the surface of an immune effector cell (called **"effector cell molecule"** herein) and to another molecule expressed on the surface of a target cell (called a **"target cell molecule"** herein). Hence, a V-C-Fc-V-C can bind to a target cell and an effector cell, as shown in the Examples below. The immune effector cell of the invention is a T cell, further rimmune effector cells disclosed can be, for example, an NK cell, a macrophage, or a neutrophil. In some embodiments the effector cell molecule is a protein included in the T cell receptor (TCR)-CD3 complex. The TCR-CD3 complex is a heteromultimer comprising a heterodimer comprising TCR$\alpha$ and TCR$\beta$ or TCR$\gamma$ and TCR8 plus various CD3 chains from among the CD3 zeta (CD3$\zeta$) chain, CD3 epsilon (CD3$\epsilon$) chain, CD3 gamma (CD3$\gamma$) chain, and CD3 delta (CD3$\delta$) chain. In some embodiments, a V-C-Fc-V-C antibody binds to a human and/or cynomolgus monkey CD3$\epsilon$ chain (the mature amino acid sequence of which is disclosed in SEQ ID NO:39), which may be part of a multimeric protein. Alternatively, an effector cell protein can be human and/or cynomolgus monkey TCR$\alpha$, TCR$\beta$, TCR$\delta$, TCRy, CD3 beta (CD3$\beta$) chain, CD3$\gamma$ chain, CD3$\delta$ chain, or CD3$\zeta$ chain.

Moreover, in some embodiments, the V-C-Fc-V-C antibody can also bind to a CD3ε chain from another species, such as mouse, rat, rabbit, new world monkey, and/or old world monkey species. Such species include, without limitation, the following mammalian species: *Mus musculus; Rattus rattus; Rattus norvegicus;* the cynomolgus monkey, Macaca *fascicularis;* the hamadryas baboon, *Papio hamadryas;* the Guinea baboon, *Papio papio;* the olive baboon, *Papio anubis;* the yellow baboon, *Papio cynocephalus;* the Chacma baboon, *Papio ursinus; Callithrix jacchus; Saguinus Oedipus;* and *Saimiri sciureus.* The mature amino acid sequence of the CD3ε chain of cynomolgus monkey is provided in SEQ ID NO:40. As is known in the art of development of protein therapeutics, having a therapeutic that can have comparable activity in humans and species commonly used for preclinical testing, such as mice and monkeys, can simplify and speed drug development. In the long and expensive process of bringing a drug to market, such advantages can be critical.

In more particular embodiments, the V-C-Fc-V-C antibody can bind to an epitope within the first 27 amino acids of a CD3ε chain, which may be a human CD3ε chain and/or a CD3ε chain from different species, optionally one of the mammalian species listed above. The epitope that the antibody binds to can be part of an amino acid sequence selected from the group consisting of SEQ ID NO:39 and SEQ ID NO:40. The epitope can contain the amino acid sequence Gln-Asp-Gly-Asn-Glu (SEQ ID NO:41). The advantages of an antibody that binds such an epitope are explained in detail in U.S. Patent Application Publication 2010/0183615. The epitope to which an antibody binds can be determined by alanine scanning, which is described in, *e.g.,* U.S. Patent Application Publication 2010/0150918.

Where a T cell is the immune effector cell, effector cell molecules, which can be effector cell proteins, to which a V-C-Fc-V-C antibody can bind include proteins that are part of a TCR-CD3 complex including, without limitation, the CD3α chain, the CD3β chain, the CD3γ, the CD3δ chain, the CD3ε chain, the CD3ζ chain, the CD3η chain, TCRα, TCRβ, TCRy, and TCRδ. Where an NK cell or a cytotoxic T cell is an immune effector cell, NKG2D. CD352, NKp46, or CD16a can be an effector cell protein. Where a CD8$^+$ T cell is an immune effector cell, 4-1BB can be an effector cell protein. Alternatively, a V-C-Fc-V-C antibody could bind to other effector cell proteins expressed on T cells, NK cells, macrophages, or neutrophils.

*Target Cells and Target Cell Molecules*

As explained above, a V-C-Fc-V-C antibody as described herein can bind to an effector cell and a target cell or a pathogen via an effector cell molecule and a target cell molecule, respectively. In some embodiments, the effector cell molecule and the target cell molecule can be proteins expressed on the surface of these cells, i.e., "effector cell proteins" and "target cell proteins." The target cell molecule is a protein or a carbohydrate (which can be attached to a protein or another kind of molecule) expressed on the surface of a cancer cell. Also disclosed are target cell moleuces that are expressed on the surface of a cell infected with a pathogen, or a cell that mediates an inflammatory or autoimmune condition. In some embodiments, the target cell molecule can be highly expressed on the target cell, although this is not required.

Where the target cell is a cancer cell, a V-C-Fc-V-C antibody as described herein can bind to a cancer cell antigen as described above. A cancer cell antigen can be a molecule displayed on a cancer cell such as a human protein or a protein from another species. For example, a V-C-Fc-V-C antibody may bind to a target cell protein from a mouse, rat, rabbit, new world monkey, and/or old world monkey species, among many others. Such species include, without limitation, the following species: *Mus musculus; Rattus rattus; Rattus norvegicus;* cynomolgus monkey, Macaca *fascicularis;* the hamadryas baboon, *Papio hamadryas;* the Guinea baboon, *Papio papio;* the olive baboon, *Papio anubis; the yellow baboon, Papio cynocephalus;* the Chacma baboon, *Papio ursinus, Callithrix jacchus, Saguinus oedipus,* and *Saimiri sciureus.*

In some disclosure, the target cell protein or molecule can be selectively expressed on an infected cell, such as, for example, a cell infected by a virus, a bacterium, or a eukaryotic pathogen. For example, in the case of an hepatitis B virus (HBV) or hepatitis C virus (HCV) infection, the target cell protein can be an envelope protein of HBV or HCV that is expressed on the surface of an infected cell. In other disclosure, the target cell protein can be gp120 encoded by human immunodeficiency virus (HIV) on HIV-infected cells. Alternatively, in some disclosure shows a V-C-Fc-V-C can bind directly to a pathogen.

In other aspects disclosed, a target cell can be a cell that mediates an autoimmune or inflammatory disease. For example, human eosinophils in asthma can be target cells, in which case, EGF-like module containing mucin-like hormone receptor (EMR1), for example, can be a target cell protein. Alternatively, excess human B cells in a systemic lupus erythematosus patient can be target cells, in which case CD19 or CD20, for example, can be a target cell protein. In autoimmune conditions, excess human Th2 T cells can be target cells, in which case CCR4 can, for example, be a target cell protein. Similarly, a target cell can be a fibrotic cell that mediates a fibrotic disease such as, for example, atherosclerosis, chronic obstructive pulmonary disease (COPD), cirrhosis, scleroderma, kidney transplant fibrosis, kidney allograft nephropathy, or a pulmonary fibrosis, including idiopathic pulmonary fibrosis and/or idiotypic pulmonary hypertension. For such fibrotic conditions, fibroblast activation protein alpha (FAP alpha) can, for example, be a target cell protein. Senescent cells can also be target cells, for example in conditions such as cancer, fibrotic disorders such as idiopathic pulmonary fibrosis, and age-related disorders, *e.g.*, Alzheimer's disease, lordokyphosis, sarcopenia, cataracts, fat loss, and dermal thinning.

### *Target Cell Cytolysis Assays*

In Example 2 below, an assay for determining whether a V-C-Fc-V-C antibody as described herein can induce cytolysis of a target cell by an immune effector cell *in vitro* is described. In this assay, the immune effector cell is a T cell. The following very similar assay can be used where the immune effector cells are NK cells.

A target cell line expressing a target cell protein of interest can be labeled with 2 $\mu$M CFSE for 15 minutes at 37 °C and then washed. An appropriate number of labeled target cells can then be incubated in one or more 96 well culture plates for 40 minutes at 4 °C, with or without a bispecific protein, a control protein, or no added protein at varying concentrations. NK cells isolated from healthy human donors can be isolated using the Miltenyi NK Cell Isolation Kit II (Miltenyi Biotec, Auburn, CA) and then added to the target cells at an Effector:Target ratio of 10:1, a ratio that can be adjusted in some cases. The NK cells, which are the immune effector cells in this assay, can be used immediately post-isolation or after overnight culture at 37°C. Plates containing tumor target cells, bispecific proteins, and immune effector cells can be cultured for 18-24 hours at 37°C with 5% $CO_2$. Appropriate control wells can also be set up. After the 18-24 hour assay period, all cells can be removed from the wells. A volume of a 7-amino-actinomycin D (7-AAD) solution equal to the volume of the content of the wells can be added to each sample. Samples can then assayed to determine the percentage of live versus dead target cells via flow cytometry as described in the Examples below.

### *Therapeutic Compositions and Uses*

The V-C-Fc-V-C antibodies described herein can be used to treat a wide variety of conditions including, for example, various forms of cancer, infections, fibrotic diseases, and/or autoimmune or inflammatory conditions.

Provided herein are pharmaceutical compositions comprising the V-C-Fc-V-C antibodies described herein. Such pharmaceutical compositions comprise a therapeutically effective amount of a V-C-Fc-V-C antibody, as described herein, plus one or more additional components such as a physiologically acceptable carrier, excipient, or diluent. Such additional components can include buffers, carbohydrates, polyols, amino acids, chelating agents, stabilizers, and/or preservatives, among many possibilities.

In some embodiments, the V-C-Fc-V-C antibodies described herein can be used to treat cell proliferative diseases, including cancer, which involve the unregulated and/or inappropriate proliferation of cells, sometimes accompanied by destruction of adjacent tissue and growth of new blood vessels, which can allow invasion of cancer cells into new areas, *i.e.* metastasis. These conditions include hematologic malignancies and solid tumor malignancies. Included within conditions treatable with the V-C-Fc-V-C antibodies described herein are non-malignant conditions that involve inappropriate cell growth, including colorectal polyps, cerebral ischemia, gross cystic disease, polycystic kidney disease, benign prostatic hyperplasia, and endometriosis. Other cell proliferative diseases that can be treated using the V-C-Fc-V-C antibodies of the present invention are, for example, cancers including mesotheliomas, squamous cell carcinomas, basel cell carcinomas, myelomas, osteosarcomas, glioblastomas, gliomas, carcinomas, adenocarcinomas, melanomas, sarcomas, acute and chronic leukemias, lymphomas, and meningiomas, Hodgkin's disease, Sezary syndrome, multiple myeloma, and lung, non-small cell lung, small cell lung, laryngeal, breast, head and neck, bladder, ovarian, skin, prostate, cervical, vaginal, gastric, renal cell, kidney, pancreatic, colorectal, endometrial, and esophageal, hepatobiliary, bone, skin, and hematologic cancers, as well as cancers of the nasal cavity and paranasal sinuses, the nasopharynx, the oral cavity, the oropharynx, the larynx, the hypolarynx, the salivary glands, the mediastinum, the stomach, the small intestine, the colon, the rectum and anal region, the ureter, the urethra, the penis, the testis, the vulva, the endocrine system, the central nervous system, and plasma cells.

Among the texts providing guidance for cancer therapy is Cancer, Principles and Practice of Oncology, 4th Edition, DeVita et al., Eds. J. B. Lippincott Co., Philadelphia, PA (1993). An appropriate therapeutic approach is chosen according to the particular type of cancer, and other factors such as the general condition of the patient, as is recognized in the pertinent field. The V-C-Fc-V-C antibodies described herein may be added to a therapy regimen using other anti-neoplastic agents in treating a cancer patient.

In some embodiments, the V-C-Fc-V-C antibodies can be administered concurrently with, before, or after a variety of drugs and treatments widely employed in cancer treatment such as, for example, chemotherapeutic agents, non-chemotherapeutic, anti-neoplastic agents, and/or radiation. For example, chemotherapy and/or radiation can occur before, during, concurrently with, and/or after any of the treatments described herein. Examples of chemotherapeutic agents are discussed above and include, but are not limited to, cisplatin, taxol, etoposide, mitoxantrone (Novantrone®), actinomycin D, cycloheximide, camptothecin (or water soluble derivatives thereof), methotrexate, mitomycin (e.g., mitomycin C), dacarbazine (DTIC), anti-neoplastic antibiotics such as adriamycin (doxorubicin) and daunomycin, and all the chemotherapeutic agents mentioned above.

The V-C-Fc-V-C antibodies described herein can also be used to treat infectious disease, for example a chronic hepatis B virus (HBV) infection, a hepatis C virus (HPC) infection, a human immunodeficiency virus (HIV) infection, an Epstein-Barr virus (EBV) infection, or a cytomegalovirus (CMV) infection, among many others. In such methods, the V-C-Fc-V-C antibodies can be used before, after, or concurrently with other treatments for these conditions.

The V-C-Fc-V-C antibodies described herein can find further use in other kinds of conditions where it is beneficial to deplete certain cell types. For example, depletion of human eosinophils in asthma, excess human B cells in systemic lupus erythematosus, excess human Th2 T cells in autoimmune conditions, or pathogen-infected cells in infectious diseases can be beneficial. Depletion of myofibroblasts or other pathological cells in fibrotic conditions such as lung fibrosis, such as idiopathic pulmonary fibrosis (IPF), or kidney or liver fibrosis is a further use of a V-C-Fc-V-C antibody.

Therapeutically effective doses of the V-C-Fc-V-C antibodies described herein can be administered. The amount of antibody that constitutes a therapeutically dose may vary with the indication treated, the weight of the patient, the calculated skin surface area of the patient. Dosing of the V-C-Fc-V-C antibodies described herein can be adjusted to achieve the desired effects. In many cases, repeated dosing may be required. For example, a V-C-Fc-V-C antibody as described herein can be dosed twice per week, once per week, once every two, three, four, five, six, seven, eight, nine, or ten weeks, or once every two, three, four, five, or six months. The amount of the V-C-Fc-V-C antibody administered on each day can be from about 0.0036 mg to about 450 mg or about 1000 mg. Alternatively, the dose can calibrated according to the estimated skin surface of a patient, and each dose can be from about $0.002 \text{ mg/m}^2$ to about $250 \text{ mg/m}^2$ or about $500 \text{ mg/m}^2$. In another alternative, the dose can be calibrated according to a patient's weight, and each dose can be from about 0. 000051 mg/kg to about 6.4 mg/kg or about 20 mg/kg.

The V-C-Fc-V-C antibodies, or pharmaceutical compositions containing these molecules, can be administered by any feasible method. Protein therapeutics will ordinarily be administered by parenteral route, for example by injection, since oral administration, in the absence of some special formulation or circumstance, would lead to hydrolysis of the protein in the acid environment of the stomach. Subcutaneous, intramuscular, intravenous, intraarterial, intralesional, or peritoneal injection are possible routes of administration. A V-C-Fc-V-C antibody can also be administered via infusion, for example intravenous or subcutaneous infusion. Topical administration is also possible, especially for diseases involving the skin. Alternatively, a V-C-Fc-V-C antibody can be administered through contact with a mucus membrane, for example by intra-nasal, sublingual, vaginal, or rectal administration or administration as an inhalant. Alternatively, certain appropriate pharmaceutical compositions comprising a V-C-Fc-V-C antibody can be administered orally.

Having described the invention in general terms above, the following examples are offered by way of illustration and not limitation.

## EXAMPLES

### Example 1: Making V-C-Fc-V-C's

Nucleic acid constructs encoding two V-C-Fc-V-C antibodies specific for CD3ε and folate receptor 1 (FOLR1) (called aFOLR1-Fc-aCD3 or aCD3-Fc-aFOLR1, depending on the order of the domains) were made using nucleic acids encoding the heavy and light chain variable regions from an anti-CD3ε antibody designated F12Q and the heavy and light chain variable regions from an anti-FOLR1 antibody designated 5G1. See Figure 2, panels A and B. In DNA constructs encoding aCD3-Fc-aFOLR1, nucleic acids encoding an anti-CD3ε half Fab fragment (heavy or light chain fragment) were upstream and nucleic acids encoding an anti-FOLR1 half Fab fragment were downstream relative to nucleic acids encoding an Fc polypeptide chain. See Figure 2, panel A. In constructs encoding aFOLR1-Fc-aCD3, nucleic acids encoding an anti-CD3ε half Fab fragment (either heavy or light chain fragment) were downstream and nucleic acids encoding an anti-FOLR1 half Fab fragment were upstream relative to nucleic acids encoding an Fc polypeptide chain. See Figure 2, panel B. The encoded Fc polypeptide chains were human IgG Fc polypeptide chains with some minor alterations as described below. Downstream from nucleic acids encoding the Fc polypeptide chain in each construct are nucleic acids encoding a linker having the amino acid sequence $(G_4S)_2$ (SEQ ID NO:36). After the nucleic acids encoding the linker are nucleic acids encoding a variable region and then either a CL or a CH1. The CH1 region is, in some cases, followed by a short portion of what is generally considered to be a hinge region, including a cysteine. Nucleic acids encoding the half Fab fragments and Fc polypeptides were generated by polymerase chain reaction (PCR) from existing constructs and joined together by PCR SOE. *See, e.g.,* Warrens et al. (1997), Gene 186: 29-35. The nucleic acid sequences encoding these two V-C-Fc-V-C's are provided in SEQ ID NOs: 6 and 8 and SEQ ID NOs: 2 and 4. The amino acid sequences encoded by these pairs of nucleic acid sequences are SEQ ID NOs: 5 and 7 (aCD3-Fc-aFOLR1) and SEQ ID NOs: 1 and 3 (aFOLR1-Fc-aCD3).

In addition, constructs encoding V-C-Fc-V-C antibodies specific for CD3ε and Human Epidermal Growth Factor Receptor (HER2) (aHER2-Fc-aCD3 and aCD3-Fc-aHER2, which are diagrammed in Figure 2, panels C and D, respectively) were made using methods like those described above from nucleic acids encoding an anti-CD3ε antibody designated F12Q and the heavy and light chain variable regions from an anti-HER2 antibody designated 7052-D3-2. Amino acid sequences of the two polypeptide chains of aHER2-Fc-aCD3 and aCD3-Fc-aHER2 are provided in SEQ ID NOs: 9 and 11 and SEQ ID NOs: 46 and 48, respectively. The nucleic acid sequences encoding these are provided in SEQ ID NOs: 10 and 12 and SEQ ID NOs: 47 and 49, respectively.

The Fc region of each of the V-C-Fc-V-C antibodies described above contained charge pair substitutions in each Fc polypeptide chain to encourage heterodimer formation. For example, SEQ ID NO:1 contains the alterations D399K and E356K (at positions 419 and 376 of SEQ ID NO:1, respectively), and SEQ ID NO:3 contains the alterations K392D and K409E (at positions 407 and 424 of SEQ ID NO:3, respectively). Polypeptide chains containing these altered Fc polypeptides will tend to form heterodimers rather than homodimers. *See, e.g.,* US Patent Application Publication 2010/0286374 and Gunasekaran et al. (2010), J. Biol. Chem. 285: 19637-19646. In addition, all of the Fc polypeptide chains specifically exemplified herein also had the alterations L234A and L235A (corresponding to positions 254 and 255 in SEQ ID NO:1), substitutions known to reduce FcγR binding.

### Example 2: Testing V-C-Fc-V-Cs for binding and biological activity

Binding of aFOLR1-Fc-aCD3 and a single chain bispecific molecule containing the same variable regions (called sc-aFOLR1-sc-aCD3) to T47D tumor cells (which express FOLR1) and purified human Pan-T cells (which express CD3ε) was analyzed by fluorescence activated cell sorting (FACS). The amino acid sequence of sc-aFOLR1-sc-aCD3ε is provided in SEQ ID NO:44. Cells were incubated for 16 hrs at 4°C in the absence or presence of 2 nM bi-specific molecule for tumor cells and 200 nM bispecific molecule for T cells. Cell binding of aFOLR1-Fc-aCD3 was detected using an allophycocyanin (APC)-labeled anti-human secondary antibody. The sc-aFOLR1-sc-aCD3 molecule, which contains a FLAG® tag, was detected using a mouse anti-FLAG® antibody followed by an APC-labeled mouse specific antibody.

FACS histograms in Figure 3 show the Mean Fluorescent Intensity (MFI) in the absence (unfilled tracing towards the left of each panel) and presence of 2 nM bispecific (for T47D cells) or 200 mM bispecific (for T cells). These data indicate that both bispecific molecules bind to T cells, which express CD3ε, and T47D tumor cells, which express FOLR1.

The following experiment was done to determine whether the aFOLR1-Fc-aCD3 could mediate T cell dependent cytolysis of target cells expressing FOLR1. Pan T effector cells isolated from healthy human donors were incubated with carboxyfluorescein succinylmidyl ester (CFSE)-labeled tumor target cells at a ratio of 10:1 in the presence and absence of various concentrations of aFOLR1-Fc-aCD3 or sc-aFOLR1-sc-aCD3. After 39-48 hours of incubation, cells were harvested, and tumor cell lysis was monitored by 7-amino-actinomycin D (7-AAD) uptake using flow cytometry. To determine the percent of total cell lysis, samples containing effector and labeled target cells without an antibody were lysed with cold 80% methanol. The percent specific lysis was calculated according to the following formula:

$$\% \text{ specific lysis} = [\% \text{ tumor lysis with bi-specific} - \% \text{ tumor lysis without bi-specific} / \% \text{ of total}$$

$$\text{lysis} - \% \text{ tumor lysis without bi-specific}] \times 100$$

Results are shown in Figure 4. Specific lysis of T47D cells (expressing about 101,000 FOLR1 sites/cell) and Cal-51 cells (expressing about 148,000 FOLR1 sites/cell), was observed with both aFOLR1-Fc-aCD3 and sc-aFOLR1-sc-aCD3. The concentration for half maximal lysis ($EC_{50}$) for sc-aFOLR1-sc-aCD3 was 0.127 pM for T47D cells and 0.130 pM for Cal-51 cells. The $EC_{50}$'s for aFOLR1-Fc-aCD3 were 105.5 pM for T47D cells and 113.9 pM for Cal-51 cells. No lysis of BT474 cells (which express undetectable, if any, levels of FOLR1) was observed with either antibody. Figure 4C. In addition, tumor target cells in the presence of aFOLR1-Fc-aCD3 or sc-aFOLR1-sc-aCD3, but in the absence of T cells, did not take up 7-AAD, indicating that T cells, which are the effector cells in this case, are required for cell death of the target cells. Data not shown. These observations suggest that the aFOLR1-Fc-aCD3 V-C-Fc-V-C is a highly specific reagent capable of inducing tumor cell lysis by T cells.

A further T cell dependent cytolysis experiment was done to determine whether the order of the segments in a V-C-Fc-V-C affected activity. The experiment was performed using Cal-51 cells as target cells, and percent specific cell lysis was determined as described above. The percent specific lysis is shown in Figure 5, and the $EC_{50}$'s of the bispecific antibodies are shown in Table 3 below.

**Table 3: $EC_{50}$'s of bispecific antibodies**

| Bispecific | $EC_{50}$ (pM) |
|---|---|
| aFOLR1-Fc-aCD3 | 60.88 |
| aCD3-Fc-aFOLR1 | 306.0 |
| sc-aFOLR1-sc-aCD3 | 0.086 |

Thus, both V-C-Fc-V-Cs have $EC_{50}$'s in the picomolar range, while sc-aFOLR1-sc-aCD3 has a lower $EC_{50}$. These observations suggest that both versions of the V-C-Fc-V-C are specific reagents capable of inducing tumor cell lysis by T cells, although one molecule, i.e., aFOLR1-Fc-aCD3, was slightly more potent than the other.

### Example 3: Testing of further bispecific molecules for binding and biological activity

Binding of aHER2-Fc-aCD3, which is described in Example 1, and a single chain bispecific molecule having the same variable regions (SEQ ID NO:42; called sc-aHER2-sc-aCD3) to SKOV-3 tumor cells, which express HER2, and to purified human Pan-T cells, which express CD3, was analyzed by FACS. Cells were incubated for 16 hrs at 4 °C in the absence and presence of a 2 nM (for the SKOV-3 cells) or a 200 nM (for the T cells) concentration of either of the two bispecific molecules. Binding of the aHER2-Fc-aCD3 was detected using an APC-labeled anti-human secondary antibody. Binding of the sc-aHER2-sc-aCD3, which has a FLAG tag, was detected using a mouse anti-FLAG antibody followed by an APC-labeled mouse IgG specific antibody.

Results are shown in Figure 6. FACS histograms show the MFI in the absence (unfilled tracing) and presence (filled tracing) of a bispecific molecule to SKOV-3 cells (top) or T cells (bottom). Results from aHER2-Fc-aCD3 and sc-aHER2-sc-aCD3 binding are shown at left and right, respectively, as indicated. These results demonstrate that both molecules bind to T cells and tumor target cells.

To assess the ability of aHER2-Fc-aCD3 and sc-aHER2-sc-aCD3 to induce T cell dependent tumor cell killing, assays were performed and percent specific cell lysis was determined as described above. Results are shown in Figure 7. Specific lysis of SKOV-3 cells (Figure 7, top panel), which express about 530,000 HER2 sites/cell, and BT474 cells (Figure 7, middle panel), which express about 300,000 HER2 sites/cell, was observed with both aHER2-Fc-aCD3 and sc-aHER2-sc-aCD3. The concentration for half maximal lysis ($EC_{50}$) was in the pM range as shown in Table 4 below.

**Table 4**

| | EC$_{50}$ (pM) | |
|---|---|---|
| Bispecific molecule | SKOV-3 cells | BT474 cells |
| aHER2-Fc-aCD3 | 165.2 | 638.0 |
| sc-aHER2-sc-aCD3 | 0.0075 | 0.086 |

There was essentially no lysis of SHP77 cells, which do not express detectable levels of HER2 (Figure 7, bottom panel). In addition, tumor target cells in the presence of either of the bispecific molecules, but in the absence of T cells, did not result in 7-AAD uptake. Data not shown. These observations indicate that both aHER2-Fc-aCD3 and sc-aHER2-sc-aCD3 are specific reagents capable of inducing tumor cell lysis in the presence of T cells.

To determine whether the order of the segments within the V-C-Fc-V-C molecule affected activity, T cell dependent cell cytolysis assays using aHER2-Fc-aCD3, aCD3-Fc-aHER2, and sc-aHER2-sc-aCD3 was performed as described above except that the target cells are JIMT-1 cells, which express about 181,000 molecules of HER2 per cell. As shown in Figure 8, aHER2-Fc-aCD3 and aCD3-Fc-aHER2 gave very similar results, having $EC_{50}$'s of 56.55 pM and 51.82 pM, respectively, while sc-aHER2-sc-aCD3 had an $EC_{50}$ of 0.203 pM. Thus, in this case, the order of the Fab fragments had little if any effect on the potency of the V-C-Fc-V-C's.

To determine the ability of the aHER2-Fc-aCD3 bispecific to activate T cells, pan T effector cells isolated from human healthy donors were labeled with CFSE and incubated with SKOV tumor cells at a ratio of 10:1 in the presence or absence of either the aHER2-Fc-aCD3 or sc-aHER2-sc-aCD3 at the concentrations indicated in Figure 9. After 72 hours, cells were harvested, and the percent of T cells that were dividing was determined by flow cytometry by monitoring of the dilution of incorporated dye with each cell cycle. Expression of CD25, a marker for activation, was detected by flow cytometry using an anti-CD25 APC-labeled antibody.

As shown in Figure 9, proliferation of T cells was observed with both aHER2-Fc-aCD3 and sc-aHER2-sc-aCD3 when incubated with HER2-expressing tumor cells, i.e., SKOV-3 cells. The $EC_{50}$'s were in the pM range as shown in Table 5 below. There was no proliferation of T cells when incubated with SHP77 cells, which do not express detectable levels of HER2. Data not shown. In addition, T cells in the presence of the aHER2-Fc-aCD3 or sc-aHER2-sc-aCD3, expressed the activation marker CD25 following incubation with HER2-expressing SKOV-3 tumor cells (Figure 7, lower panel), but not with the HER2-negative tumor line, SHP77 (data not shown). These observations suggest that the V-C-Fc-V-C is a specific reagent capable of inducing T cell activation and proliferation.

**Table 5**

| Bispecific molecule | EC$_{50}$ (pM) | |
|---|---|---|
| | % of T cells that are dividing | % CD25$^+$ T cells |
| aHER2-Fc-aCD3 | 76.72 | 79.32 |
| sc-aHER2-sc-aCD3 | 0.330 | 0.136 |

### Example 4: Pharmacokinetic properties of a V-C-Fc-V-C

The following experiment was done to evaluate the single dose pharmacokinetics of a V-C-Fc-V-C molecule (aHER-Fc-aCD3) vs. single chain bispecific molecule (sc-aHER2-sc-aCD3) containing two scFv's linked in tandem. The single chain molecule contained and an anti-HER2 scFv followed by an anti-CD3 scFv, and its amino acid sequence is provided in SEQ ID NO:42. Each polypeptide chain of the V-C-Fc-V-C contained an anti HER2 variable region (either VH or VL) at its N-terminus and an anti-CD3 variable region (either VH or VL) following the CH3 domain. The sequences of the two polypeptide chains are provided in SEQ ID NOs:9 and 11. The same anti-HER2 and anti-CD3 variable regions were included in both sc-aHER2-sc-aCD3 and aHER2-Fc-aCD3. The pharmacokinetic properties of these antibodies were compared following intravenous and subcutaneous bolus administration in male NOD.SCID mice (Harlan, Livermore, CA).

The antibodies were dosed intravenously (see Figure 10) via the lateral tail vein or subcutaneously (see Figure 11) under the skin over the shoulders at 1 mg/kg. Serial bleeds of approximately 0.100 mL of whole blood were collected via retro-orbital sinus puncture for each time point. Upon clotting of whole blood the samples were processed to obtain serum (yielding about 0.40 mL per sample). Serum samples were analyzed by immunoassay to determine the serum concentrations of the antibodies. Serum samples were collected at 0, 0.5, 2, 8, 24, 72, 120, 168, 240, 312, 384, and 480 hours. Serum samples were maintained at -70°C ($\pm$10°C) prior to analysis. Pharmacokinetic parameters were estimated from serum concentrations using NCA (Phoenix 6.3, Sunnyvale, CA). Results are shown in Figures 10 and 11.

As shown in Figures 10 and 11, a HER2-Fc-aCD3 was relatively stable *in vivo,* whereas the scFc was rapidly eliminated. Thus, the V-C-Fc-V-C has improved pharmacokinetic properties relative to the single chain antibody.

### Example 5: Making V-C-Fc-V-Cs containing protease cleavage sites and control molecules

As a control, a heterodimeric bispecific antibody that binds to both CD3 and HER2 (P136797.3; called "aCD3/HER2 heterodimer") was constructed. One polypeptide comprises a VH region from an antibody that binds to CD3, followed by a linker, followed by a VL region from an antibody that binds to HER2, followed by a CH1 region, followed by a linker and an Fc polypeptide chain from a human IgG1 antibody. The sequence of this polypeptide is provided in SEQ ID NO:60. The other polypeptide chain of this heterodimer comprises a VH region from an antibody that binds to HER2, followed by a linker, followed by a VL region from an antibody that binds to CD3, followed by a CL region, followed by a linker and an Fc polypeptide chain from a human IgG1 antibody. The sequence of this polypeptide is provided in SEQ ID NO:61. DNAs encoding these polypeptides were introduced into HEK-293 cells, and the protein was recovered from the culture medium.

V-C-Fc-V-Cs containing furin or matrix metalloproteinase 2 (MMP2) cleavage sites were made by the same methods, i.e., PCR-SOE of existing nucleic acids, including insertion of protease cleavage sites and linkers via the PCR primers. See Example 1. A furin cleavage site (RRRRRR (SEQ ID NO:52)) was inserted downstream from the Fc polypeptide chain in one polypeptide chain of one V-C-Fc-V-C (aHer2-Fc-Fur-aCD3, comprising SEQ ID NOs:11 and 62), and an MMP2 cleavage site (GPLGIAGQ (SEQ ID NO:66)) was inserted downstream from the Fc polypeptide chain in one polypeptide chain of another V-C-Fc-V-C (aHer2-Fc-MMP2-aCD3, comprising SEQ ID NOs:11 and 64). These proteins were produced by transfecting DNA encoding them into HEK-293 cells, culturing the transfected cells, and recovering the protein from the cell culture supernatant. As is known in the art, HEK-293 cells express furin protease intracellularly, which has been observed to cleave recombinant proteins produced in HEK-293 cells. *See, e.g.,* Wu et al. (2003), J. Biol. Chem. 278: 25847-25852. Hence, it was expected that the V-C-Fc-V-C comprising a furin cleavage site would be cleaved. As expected, Western blots of polyacrylamide gels run under reducing conditions revealed that the aHer2-Fc-Fur-aCD3 protein recovered from the HEK-293 cells was cleaved, whereas the aHer2-Fc-MMP2-aCD3 protein was not. Data not shown.

### Example 6: Testing V-C-Fc-V-Cs containing protease cleavage site for binding and cytolytic activity

The aHER2-Fc-Fur-aCD3 and aHER2-Fc-MMP-aCD3 V-C-Fc-V-Cs were tested for binding to pan T cells (which express CD3) or T47D cells (which express HER2) as described in Example 2. The results are shown in Figures 12A, 12B, and 13. The aHER2-Fc-Fur-aCD3 V-C-Fc-V-C, which was cleaved at the furin site (see above), binds to pan T cells almost as well as the positive control aCD3/HER2 heterodimer and much better than the aHER2-Fc-aCD3 V-C-Fc-V-Cs that do not contain protease cleavage sites. Figure 12A

. The aHER2-Fc-MMP-aCD3 V-C-Fc-V-C, which was not cleaved, is similar to the aHER2-Fc-aCD3 V-C-Fc-V-Cs in its binding to pan T cells. Figure 12A,. These results indicate that cleavage of one polypeptide chain of a V-C-Fc-V-C in the linker between the CH3 region and the following variable region, increases binding of the pair of variable regions following the CH3 regions to their target, in this case, CD3. As shown in Figure 12B, all molecules tested had similar binding properties with regard to HER2. Together these results indicate that cleavage at a protease site downstream from one of the CH3 regions of a V-C-Fc-V-C facilitates binding of the two variable regions downstream from the CH3 regions to their target, but has no effect on the binding properties of the amino-terminal variable regions.

The same antibodies were tested for their ability to cause lysis of HER2-expressing cells using the methods described in Example 2, using T47D cells as target cells and pan T cells as effector cells and using an effector:target ratio of 10:1. Only the positive control, aCD3/HER2 heterodimer, showed substantial amounts of target cell lysis, although the aHER2-Fc-Fur-aCD3 antibody showed some indication of lysis at the highest concentration tested. Figure 13. Thus, while cleavage of one polypeptide chain of a V-C-Fc-V-C downstream from the Fc polypeptide chain increased binding of the more C-terminal variable regions to their antigen such that it was comparable to that of a control molecule, cleavage did not increase cytolytic activity of a cleaved V-C-Fc-V-C to be comparable to that of a control molecule. However, other linkers and/or protease cleavage sites could possibly cause substantial increases in both binding and cytolytic activity.

**Claims**

1. An antibody comprising two polypeptide chains,
   the first polypeptide chain comprising the following regions in the following order in an N- to C-terminal direction: V-1---C-1---hinge---CH2-1---CH3-1---V-2---C-2; and
   the second polypeptide chain comprising the following regions in the following order in an N-to C-terminal direction: V-3---C-3---hinge---CH2-2---CH3-2---V-4---C-4;
   wherein the V-1 to V-4 are immunoglobulin variable regions;
   wherein the C-1 to C-4 are either a heavy chain constant region 1 (CH1) or a light chain constant regions (CL), and if C-1 is a CH1, then C-3 is a CL and vice versa, and if C-2 is a CH1, then C-4 is a CL and vice versa; and
   wherein the CH2-1 and CH2-2 are each a heavy chain constant region 2 and the CH3-1 and CH3-2 are each a heavy chain constant region 3,
   wherein either the linker between CH3-1 and V-2 or the linker between CH3-2 and V-4 comprises a cleavage site for a protease, wherein the protease is preferably a furin or a matrix metalloproteinase, wherein

   a) the V-1 and the V-3 can bind to an immune effector cell when they are part of an IgG and/or scFv antibody, and the V-2 and the V-4 can bind to a target cell when they are part of an IgG and/or scFv antibody; or
   b) the V-2 and the V-4 can bind to an immune effector cell when they are part of an IgG and/or scFv antibody, and the V-1 and the V-3 can bind to a target cell when they are part of an IgG and/or scFv antibody; and

   wherein the immune effector cell is a human CD3 epsilon expressing T cell, and the target cell is a cancer cell.

2. The antibody of claim 1, wherein

   a) the V-1 is a heavy chain variable region (VH), the C-1 is a CH1, the V-2 is a VH, the C-2 is a CH1, the V-3 is a light chain variable region (VL), the C-3 is a CL, the V-4 is a VL, and the C-4 is a CL;
   b) the V-1 is a VH, the C-1 is a CH1, the V-2 is a VL, the C-2 is a CL, the V-3 is a VL, the C-3 is a CL, the V-4 is a VH, and the C-4 is a CH1;
   c) the V-1 is a VH, the C-1 is a CH1, the V-2 is a VH, the C-2 is a CL, the V-3 is a VL, the C-3 is a CL, the V-4 is a VL, and the C-4 is a CH1;
   d) the V-1 is a VH, the C-1 is a CL, the V-2 is a VH, the C-2 is a CH1, the V-3 is a VL, the C-3 is a CH1, the V-4 is a VL, and the C-4 is a CL;
   e) the V-1 is a VL, the C-1 is a CH1, the V-2 is a VH, the C-2 is a CH1, the V-3 is a VH, the C-3 is a CL, the V-4 is a VL, and the C-4 is a CL;
   f) the V-1 is a VL, the C-1 is a CH1, the V-2 is a VL, the C-2 is a CH1, the V-3 is a VH, the C-3 is a CL, the V-4 is a VH, and the C-4 is a CL;
   g) the V-1 is a VH, the C-1 is a CH1, the V-2 is a VL, the C-2 is a CH1, the V-3 is a VL, the C-3 is a CL, the V-4 is a VH, and the C-4 is a CL; or

h) the V-1 is a VL, the C-1 is a CH1, the V-2 is a VH, the C-2 is a CL, the V-3 is a VH, the C-3 is a CL, the V-4 is a VL, and the C-4 is a CH1.

3. The antibody of claim 1 or 2, wherein the first polypeptide chain comprises a linker between the CH3-1 and the V-2 and the second polypeptide chain comprises a linker between the CH3-2 and the V-4.

4. The antibody of claim 3, wherein the linkers between the CH3-1 and the V-2 and between the CH3-2 and the V-4 are each from 5 to 35 amino acids, preferably from 5 to 30 amino acids, long.

5. The antibody of any one of claims 1 to 4, wherein the CH3-1 and the CH3-2 have different amino acid sequences and comprise charge pair substitutions.

6. The antibody of claim 5, wherein
the CH3-1 comprises the charge pair substitutions K409D or K409E and K392D or K392E and the CH3-2 comprises the charge pair substitutions D399K or D399R and D356K or D356R; or the CH3-2 comprises the charge pair substitutions K409D or K409E and K392D or K392E, and the CH3-1 comprises the charge pair substitutions D399K or D399R and D356K or D356R.

7. The antibody of any one of claims 1 to 6, wherein the C-1 to C4, the hinge regions, and the CH2-1, CH2-2, CH3-1, and CH3-2 are human IgG constant regions or human lambda or kappa constant regions.

8. The antibody of claim 7, wherein the human IgG constant regions are human IgG1 constant regions, human IgG2 constant regions or human IgG4 constant regions.

9. The antibody of any one of claims 1 to 8, wherein

a) the first and second polypeptide chains comprise one or more alterations that reduce Fc gamma receptor (FcγR) binding, preferably alterations selected from the group consisting of: L234A, L235A, and any substitution at N297; or
b) the first and second polypeptide chains comprise one or more alterations that enhance(s) ADCC, preferably an insertion of the amino acid sequence of any one of SEQ ID NOs: 13-24 between positions 384 and 385 according to the EU numbering system as shown in Table 2 in CH3-1 and CH3-2.

10. An antibody comprising a first polypeptide chain having the following formula: V-1---L-3---C-1---L-4---hinge---CH2-1---CH3-1---L-1---V-2---L-5---C-2 and a second polypeptide chain having the following formula: V-3-L6---C-3---L7---hinge---CH2-2---CH3-2---L-2---V-4---L-8---C-4, wherein the V-1, V-2, V-3, and V-4 are immunoglobulin variable regions having different amino acid sequences,
wherein either the V-1 or the V-3 is a VH, and if the V-1 is a VH, then the V-3 is a VL, and if the V-3 is a VH, then the V-1 is a VL,
wherein either the V-2 or the V-4 is a VH, and if the V-2 is a VH, then the V-4 is a VL, and if the V-4 is a VH, then the V-2 is a VL,
wherein the L-1, L-2, L-3, L-4, L-5, L-6, L-7, and L-8 are linkers,
wherein either the C-1 or the C-3 is a CH1, and if the C-1 is a CH1, then the C-3 is a CL, and if the C-3 is a CH1, then the C-1 is a CL,
wherein either the C-2 or the C-4 is a CH1, and if the C-2 is a CH1, then the C-4 is a CL, and if the C-4 is a CH1, then the C-2 is a CL,
wherein the L-1 comprises a protease cleavage site and the L-2 does not, or vice versa,
wherein

a) the V-1 and the V-3 can bind to an immune effector cell when they are part of an IgG and/or scFv antibody, and the V-2 and the V-4 can bind to a target cell when they are part of an IgG and/or scFv antibody; or
b) the V-2 and the V-4 can bind to an immune effector cell when they are part of an IgG and/or scFv antibody, and the V-1 and the V-3 can bind to a target cell when they are part of an IgG and/or scFv antibody; and

wherein the immune effector cell is a human CD3 epsilon expressing T cell, and the target cell is a cancer cell.

11. The antibody of claim 10, wherein the first and the second polypeptide chains each comprise a charge pair substitution.

**12.** The antibody of claim 11, wherein
the CH3-1 comprises the charge pair substitutions K409D or K409E and K392D or K392E and the CH3-2 comprises the charge pair substitutions D399K or D399R and E356K or E356R; or the CH3-2 comprises the charge pair substitutions K409D or K409E and K392D or K392E, and the CH3-1 comprises the charge pair substitutions D399K or D399R and D356K or D356R.

**13.** The antibody of any one of claims 10 to 12, wherein the first and second polypeptide chains comprise one or more alterations that inhibit FcγR binding, preferably wherein the first and second polypeptide chains each comprise at least one alteration selected from the group consisting of: L234A, L235A, and any substitution at N297.

**14.** A pharmaceutical composition comprising the antibody of any one of claims 1 to 13.

**15.** One or more nucleic acid(s) encoding the antibody of any one of claims 1 to 13.

**16.** One or more vector(s) containing the nucleic acid(s) of claim 15.

**17.** A host cell containing the nucleic acid(s) of claim 15 or the vector(s) of claim 16.

**18.** A method of making an antibody comprising culturing the host cell of claim 17 under conditions such that the nucleic acid(s) is (are) expressed, and recovering the antibody from the cell mass or the culture medium.

**19.** The antibody of any one of claims 1 to 13 for use in the treatment of a cancer, an infectious disease, an autoimmune disease, asthma, or a fibrotic disease.


**Patentansprüche**

**1.** Ein Antikörper, der zwei Polypeptidketten umfasst,
die erste Polypeptidkette umfasst die folgenden Regionen in der folgenden Reihenfolge in N- zu C-terminaler Richtung:
V-1---C-1---Gelenk---CH2-1---CH3-1---V-2---C-2; und
die zweite Polypeptidkette umfasst die folgenden Regionen in der folgenden Reihenfolge in N- zu C-terminaler Richtung:
V-3---C-3---Gelenk---CH2-2---CH3-2---V-4---C-4;
wobei die V-1 bis V-4 variable Immunglobulinregionen sind;
wobei die C-1 bis C-4 entweder eine konstante Region der schweren Kette 1 (CH1) oder eine konstante Region der leichten Kette (CL) sind, und wenn C-1 ein CH1 ist, dann ist C-3 ein CL und umgekehrt, und wenn C-2 ein CH1 ist, dann ist C-4 ein CL und umgekehrt; und
wobei CH2-1 und CH2-2 jeweils eine konstante Region 2 der schweren Kette und CH3-1 und CH3-2 jeweils eine konstante Region 3 der schweren Kette sind,
wobei entweder der Linker zwischen CH3-1 und V-2 oder der Linker zwischen CH3-2 und V-4 eine Spaltstelle für eine Protease umfasst, wobei die Protease vorzugsweise eine Furin- oder eine Matrix-Metalloproteinase ist, wobei

a) die V-1 und die V-3 an eine Immuneffektorzelle binden können, wenn sie Teil eines IgG- und/oder scFv-Antikörpers sind, und die V-2 und die V-4 an eine Zielzelle binden können, wenn sie Teil eines IgG- und/oder scFv-Antikörpers sind; oder
b) die V-2 und die V-4 an eine Immuneffektorzelle binden können, wenn sie Teil eines IgG- und/oder scFv-Antikörpers sind, und die V-1 und die V-3 an eine Zielzelle binden können, wenn sie Teil eines IgG- und/oder scFv-Antikörpers sind; und

wobei die Immuneffektorzelle eine humanes CD3-Epsilon exprimierende T-Zelle ist und die Zielzelle eine Krebszelle ist.

**2.** Der Antikörper nach Anspruch 1, wobei

a) die V-1 eine variable Region der schweren Kette (VH) ist, die C-1 eine CH1 ist, die V-2 eine VH ist, die C-2 eine CH1 ist, die V-3 eine variable Region der leichten Kette (VL) ist, die C-3 eine CL ist, die V-4 eine VL ist und die C-4 eine CL ist;

b) die V-1 eine VH ist, die C-1 eine CH1 ist, die V-2 eine VL ist, die C-2 eine CL ist, die V-3 eine VL ist, die C-3 eine CL ist, die V-4 eine VH ist, und die C-4 eine CH1 ist;

c) die V-1 eine VH ist, die C-1 eine CH1 ist, die V-2 eine VH ist, die C-2 eine CL ist, die V-3 eine VL ist, die C-3 eine CL ist, die V-4 eine VL ist und die C-4 eine CH1 ist;

d) die V-1 eine VH ist, die C-1 eine CL ist, die V-2 eine VH ist, die C-2 eine CH1 ist, die V-3 eine VL ist, die C-3 eine CH1 ist, die V-4 eine VL ist, und die C-4 eine CL ist;

e) die V-1 eine VL ist, die C-1 eine CH1 ist, die V-2 eine VH ist, die C-2 eine CH1 ist, die V-3 eine VH ist, die C-3 eine CL ist, die V-4 eine VL ist, und die C-4 eine CL ist;

f) die V-1 eine VL ist, die C-1 eine CH1 ist, die V-2 eine VL ist, die C-2 eine CH1 ist, die V-3 eine VH ist, die C-3 eine CL ist, die V-4 eine VH ist, und die C-4 eine CL ist;

g) die V-1 eine VH ist, die C-1 eine CH1 ist, die V-2 eine VL ist, die C-2 eine CH1 ist, die V-3 eine VL ist, die C-3 eine CL ist, die V-4 eine VH ist und die C-4 eine CL ist; oder

h) die V-1 eine VL ist, die C-1 eine CH1 ist, die V-2 eine VH ist, die C-2 eine CL ist, die V-3 eine VH ist, die C-3 eine CL ist, die V-4 eine VL ist und die C-4 eine CH1 ist.

3. Der Antikörper nach Anspruch 1 oder 2, wobei die erste Polypeptidkette einen Linker zwischen der CH3-1 und der V-2 und die zweite Polypeptidkette einen Linker zwischen der CH3-2 und der V-4 umfasst.

4. Der Antikörper nach Anspruch 3, wobei die Linker zwischen der CH3-1 und der V-2 und zwischen der CH3-2 und der V-4 jeweils 5 bis 35 Aminosäuren, vorzugsweise 5 bis 30 Aminosäuren, lang sind.

5. Der Antikörper nach einem der Ansprüche 1 bis 4, wobei die CH3-1 und die CH3-2 unterschiedliche Aminosäuresequenzen haben und Ladungspaar-Substitutionen umfassen.

6. Der Antikörper nach Anspruch 5, wobei
die CH3-1 die Ladungspaar-Substitutionen K409D oder K409E und K392D oder K392E umfasst und die CH3-2 die Ladungspaar-Substitutionen D399K oder D399R und D356K oder D356R umfasst; oder
die CH3-2 die Ladungspaar-Substitutionen K409D oder K409E und K392D oder K392E umfasst und das CH3-1 die Ladungspaar-Substitutionen D399K oder D399R und D356K oder D356R umfasst.

7. Der Antikörper nach einem der Ansprüche 1 bis 6, wobei die C-1 bis C4, die Gelenkregionen und die CH2-1, CH2-2, CH3-1 und CH3-2 Regionen mit konstantem humanen IgG oder Regionen mit konstantem humanen Lambda oder Kappa sind.

8. Der Antikörper nach Anspruch 7, wobei die konstanten Regionen des humanen IgG humane IgG1 konstante Regionen, humane IgG2 konstante Regionen oder humane IgG4 konstante Regionen sind.

9. Der Antikörper nach einem der Ansprüche 1 bis 8, wobei

a) die erste und zweite Polypeptidkette eine oder mehrere Veränderungen umfassen, die die Bindung des Fc-Gammarezeptors (FcγR) inhibieren, vorzugsweise Veränderungen ausgewählt aus der Gruppe bestehend aus: L234A, L235A und jegliche Substitution an N297; oder

b) die erste und die zweite Polypeptidkette eine oder mehrere Veränderungen umfassen, die die ADCC verstärken, vorzugsweise eine Insertion der Aminosäuresequenz einer der SEQ ID NOs: 13-24 zwischen den Positionen 384 und 385 gemäß dem EU-Nummerierungssystem, wie in Tabelle 2 in CH3-1 und CH3-2 gezeigt.

10. Ein Antikörper, umfassend eine erste Polypeptidkette mit der folgenden Formel: V-1---L-3---C-1---L-4---Gelenk---CH2-1---CH3-1---L-1---V-2---L-5---C-2 und eine zweite Polypeptidkette mit der folgenden Formel: V-3-L6---C-3---L7---Gelenk---CH2-2-CH3-2---L-2---V-4---L-8---C-4,
wobei die V-1, V-2, V-3 und V-4 variable Immunglobulinregionen mit unterschiedlichen Aminosäuresequenzen sind,
wobei entweder die V-1 oder die V-3 eine VH ist, und wenn die V-1 eine VH ist, dann ist die V-3 eine VL, und wenn die V-3 eine VH ist, dann ist die V-1 eine VL,
wobei entweder die V-2 oder die V-4 eine VH ist, und wenn die V-2 eine VH ist, dann ist die V-4 eine VL, und wenn die V-4 eine VH ist, dann ist die V-2 eine VL,
wobei die L-1, L-2, L-3, L-4, L-5, L-6, L-7 und L-8 Linker sind,
wobei entweder die C-1 oder die C-3 eine CH1 ist, und wenn die C-1 eine CH1 ist, dann ist die C-3 eine CL, und wenn die C-3 eine CH1 ist, dann ist die C-1 eine CL, wobei entweder die C-2 oder die C-4 eine CH1 ist, und wenn die C-2 eine CH1 ist, dann ist die C-4 eine CL, und wenn die C-4 eine CH1 ist, dann ist die C-2 eine CL, wobei der L-1 eine Protease-Spaltstelle umfasst und der L-2 nicht, oder umgekehrt, wobei

a) die V-1 und die V-3 an eine Immuneffektorzelle binden können, wenn sie Teil eines IgG- und/oder scFv-Antikörpers sind, und die V-2 und die V-4 an eine Zielzelle binden können, wenn sie Teil eines IgG- und/oder scFv-Antikörpers sind; oder

b) die V-2 und die V-4 an eine Immuneffektorzelle binden können, wenn sie Teil eines IgG- und/oder scFv-Antikörpers sind, und die V-1 und die V-3 an eine Zielzelle binden können, wenn sie Teil eines IgG- und/oder scFv-Antikörpers sind; und

wobei die Immuneffektorzelle eine humane CD3-Epsilon exprimierende T-Zelle ist und die Zielzelle eine Krebszelle ist.

11. Der Antikörper nach Anspruch 10, wobei die erste und die zweite Polypeptidkette jeweils eine Ladungspaar-Substitution umfassen.

12. Der Antikörper nach Anspruch 11, wobei
die CH3-1 die Ladungspaar-Substitutionen K409D oder K409E und K392D oder K392E umfasst und die CH3-2 die Ladungspaar-Substitutionen D399K oder D399R und D356K oder D356R umfasst; oder
die CH3-2 die Ladungspaar-Substitutionen K409D oder K409E und K392D oder K392E umfasst und das CH3-1 die Ladungspaar-Substitutionen D399K oder D399R und D356K oder D356R umfasst.

13. Der Antikörper nach einem der Ansprüche 10 bis 12, wobei die erste und die zweite Polypeptidkette eine oder mehrere Veränderungen umfassen, die die Bindung des Fc-Gammarezeptors (FcγR) inhibieren, vorzugsweise wobei die erste und die zweite Polypeptidkette jeweils Veränderungen umfasst, die ausgewählt sind aus der Gruppe bestehend aus: L234A, L235A und jeglicher Substitution an N297.

14. Eine Pharmazeutische Zusammensetzung umfassend den Antikörper nach einem der Ansprüche 1 bis 13.

15. Eine oder mehrere Nukleinsäure(n), die für den Antikörper nach einem der Ansprüche 1 bis 13 kodieren.

16. Ein oder mehrere Vektor(en), die die Nukleinsäure(n) nach Anspruch 15 enthalten.

17. Eine Wirtszelle umfassend die Nukleinsäure(n) nach Anspruch 15 oder den/die Vektor(en) nach Anspruch 16.

18. Ein Verfahren zur Herstellung eines Antikörpers umfassend das Kultivieren der Wirtszelle nach Anspruch 17 unter Bedingungen, sodass die Nukleinsäure(n) exprimiert wird/werden, und gewinnen des Antikörpers von der Zellmasse oder dem Kulturmedium

19. Der Antikörper nach einem der Ansprüche 1 bis 13 zur Verwendung in der Behandlung von Krebs, einer Infektionskrankheit, einer Autoimmunkrankheit, Asthma oder einer fibrotischen Krankheit.

**Revendications**

1. Anticorps comprenant deux chaînes polypeptidiques,
la première chaîne polypeptidique comprenant les régions suivantes dans l'ordre suivant dans une direction N- à C-terminale :
V-1-C-1-charnière-CH2-1-CH3-1-V-2-C-2; et
la seconde chaîne polypeptidique comprenant les régions suivantes dans l'ordre suivant dans une direction N- à C-terminale:
V-3-C-3 charnière-CH2-2-CH3-2-V-4-C-4;
dans lequel les V-1 à V-4 sont des régions variables d'immunoglobuline; dans lequel les C-1 à C-4 sont soit une région constante de chaîne lourde 1 (CHI) soit une région constante de chaîne légère (CL), et si C-1 est une CH1, alors C-3 est une CL et vice versa, et si C-2 est une CH1, alors C-4 est une CL et vice versa; et
dans lequel les CH2-1 et CH2-2 sont chacun une région constante de chaîne lourde 2 et les CH3-1 et CH3-2 sont chacun une région constante de chaîne lourde 3,

dans lequel soit le lieur entre CH3-1 et V-2, soit le lieur entre CH3-2 et V-4 comprend un site de clivage pour une protéase, dans lequel la protéase est de préférence une furine ou une métalloprotéinase matricielle, dans lequel

> a) la V-1 et la V-3 peuvent se lier à une cellule effectrice immunitaire lorsqu'ils font partie d'un anticorps IgG et/ou scFv, et la V-2 et la V-4 peuvent se lier à une cellule cible lorsqu'ils font partie d'un anticorps IgG et/ou scFv; ou
> b) la V-2 et la V-4 peuvent se fixer à une cellule effectrice immunitaire lorsqu'ils font partie d'un anticorps IgG et/ou scFv, et la V-1 et la V-3 peuvent se fixer à une cellule cible lorsqu'ils font partie d'un anticorps IgG et/ou scFv; et

dans laquelle la cellule effectrice immunitaire est une cellule T exprimant le CD3 epsilon humain, et la cellule cible est une cellule cancéreuse.

2. Anticorps selon la revendication 1, dans lequel

> a) la V-1 est une région variable de la chaîne lourde (VH), la C-1 est une CH1, la V-2 est une VH, la C-2 est une CH1, la V-3 est une région variable de la chaîne légère (VL), la C-3 est une CL, la V-4 est une VL, et la C-4 est une CL;
> b) la V-1 est une VH, la C-1 est une CH1, la V-2 est une VL, la C-2 est une CL, la V-3 est une VL, la C-3 est une CL, la V-4 est une VH, et la C-4 est une CH1;
> c) la V-1 est une VH, la C-1 est une CH1, la V-2 est une VH, la C-2 est une CL, la V-3 est une VL, la C-3 est une CL, la V-4 est une VL et la C-4 est une CH1;
> d) la V-1 est une VH, la C-1 est une CL, la V-2 est une VH, la C-2 est une CH1, la V-3 est une VL, la C-3 est une CH1, la V-4 est une VL, et la C-4 est une CL;
> e) la V-1 est une VL, la C-1 est une CH1, la V-2 est une VH, la C-2 est une CH1, la V-3 est une VH, la C-3 est une CL, la V-4 est une VL et la C-4 est une CL;
> f) la V-1 est une VL, la C-1 est une CH1, la V-2 est une VL, la C-2 est une CH1, la V-3 est une VH, la C-3 est une CL, la V-4 est une VH et la C-4 est une CL;
> g) la V-1 est une VH, la C-1 est une CH1, la V-2 est une VL, la C-2 est une CH1, la V-3 est une VL, la C-3 est une CL, la V-4 est une VH et la C-4 est une CL; ou
> h) la V-1 est une VL, la C-1 est une CH1, la V-2 est une VH, la C-2 est une CL, la V-3 est une VH, la C- 3 est une CL, la V-4 est une VL et la C-4 est une CH1.

3. Anticorps selon la revendication 1 ou 2, dans lequel la première chaîne polypeptidique comprend un lieur entre la CH3-1 et la V-2 et la seconde chaîne polypeptidique comprend un lieur entre la CH3-2 et la V-4.

4. Anticorps selon la revendication 3, dans lequel les lieurs entre la CH3-1 et la V-2 et entre la CH3-2 et la V-4 ont chacun une longueur de 5 à 35 acides aminés, de préférence de 5 à 30 acides aminés.

5. Anticorps selon l'une quelconque des revendications 1 à 4, dans lequel la CH3-1 et la CH3-2 ont des séquences d'acides aminés différentes et comprennent des substitutions de paires de charges.

6. Anticorps selon la revendication 5, dans lequel
la CH3-1 comprend les substitutions de paires de charges K409D ou K409E et K392D ou K392E et la CH3-2 comprend les substitutions de paires de charges D399K ou D399R et D356K ou D356R ; ou la CH3-2 comprend les substitutions de paires de charges K409D ou K409E et K392D ou K392E, et la CH3-1 comprend les substitutions de paires de charges D399K ou D399R et D356K ou D356R.

7. Anticorps selon l'une quelconque des revendications 1 à 6, dans lequel les C-1 à C4, les régions charnières et les CH2-1, CH2-2, CH3-1 et CH3-2 sont des régions constantes d'IgG humaines ou des régions constantes lambda ou kappa humaines.

8. Anticorps selon la revendication 7, dans lequel les régions constantes des IgG humaines sont des régions constantes des IgGI humaines, des régions constantes des IgG2 humaines ou des régions constantes des IgG4 humaines.

9. Anticorps selon l'une quelconque des revendications 1 à 8, dans lequel

> a) les première et seconde chaînes polypeptidiques comprennent une ou plusieurs altérations qui réduisent la liaison au récepteur Fc gamma (FcγR), de préférence des altérations choisies dans le groupe constitué par: L234A, L235A, et toute substitution au niveau de N297; ou

b) les première et seconde chaînes polypeptidiques comprennent une ou plusieurs altérations qui améliorent l'ADCC, de préférence une insertion de la séquence d'acides aminés de l'une quelconque des SEQID NO : 13-24 entre les positions 384 et 385 selon le système de numérotation de l'UE comme indiqué dans le tableau 2 dans CH3-1 et CH3-2.

**10.** Anticorps comprenant une première chaîne polypeptidique ayant la formule suivante V-1-L-3-C-1-L-4--charnière-CH2-1-CH3-1-L-I-V-2-L-5-C-2 et une seconde chaîne polypeptidique ayant la formule suivante: V-3-L6-C-3-L7-charnière-CH2-2-CH3-2-L-2-V-4-L-8-C-4, dans laquelle les V-1, V-2, V-3 et V-4 sont des régions variables d'immunoglobuline ayant des séquences d'acides aminés différentes,

dans lequel soit la V-1 soit la V-3 est une VH, et si la V-1 est une VH, alors la V-3 est une VL, et si la V-3 est une VH, alors la V-1 est une VL,

dans lequel soit la V-2 soit la V-4 est une VH, et si la V-2 est une VH, alors la V-4 est une VL, et si la V-4 est une VH, alors la V-2 est une VL,

dans lequel les L-I, L-2, L-3, L-4, L-5, L-6, L-7 et L-8 sont des lieurs, où soit la C-1 soit la C-3 est une CH1, et si la C-1 est une CH1, alors la C-3 est une CL, et si la C-3 est une CH1, alors la C-1 est une CL,

dans lequel soit la C-2 soit la C-4 est une CH1, et si la C-2 est une CH1, alors la C-4 est une CL, et si la C-4 est une CH1, alors la C-2 est une CL, où le L-I comprend un site de clivage de protéase et le L-2 ne comprend pas, ou vice versa, dans lequel

a) la V-1 et la V-3 peuvent se lier à une cellule effectrice immunitaire lorsqu'ils font partie d'un anticorps IgG et/ou scFv, et la V-2 et la V-4 peuvent se lier à une cellule cible lorsqu'ils font partie d'un anticorps IgG et/ou scFv; ou

b) la V-2 et la V-4 peuvent se fixer à une cellule effectrice immunitaire lorsqu'ils font partie d'un anticorps IgG et/ou scFv, et la V-1 et la V-3 peuvent se fixer à une cellule cible lorsqu'ils font partie d'un anticorps IgG et/ou scFv; et

dans laquelle la cellule effectrice immunitaire est une cellule T exprimant le CD3 epsilon humain, et la cellule cible est une cellule cancéreuse.

**11.** Anticorps selon la revendication 10, dans lequel la première et la seconde chaînes polypeptidiques comprennent chacune une substitution de paire de charges.

**12.** Anticorps selon la revendication 11, dans lequel
la CH3-1 comprend les substitutions de paires de charges K409D ou K409E et K392D ou K392E et
la CH3-2 comprend les substitutions de paires de charges D399K ou D399R et E356K ou E356R; ou
la CH3-2 comprend les substitutions de paires de charges K409D ou K409E et K392D ou K392E, et
la CH3-1 comprend les substitutions de paires de charges D399K ou D399R et D356K ou D356R.

**13.** Anticorps selon l'une quelconque des revendications 10 à 12, dans lequel les première et seconde chaînes polypeptidiques comprennent une ou plusieurs altérations qui inhibent la liaison FcγR, de préférence dans lequel les première et seconde chaînes polypeptidiques comprennent chacune au moins une altération choisie dans le groupe constitué par : L234A, L235A, et toute substitution au niveau de N297.

**14.** Composition pharmaceutique comprenant l'anticorps de l'une quelconque des revendications 1 à 13.

**15.** Un ou plusieurs acide(s) nucléique(s) codant pour l'anticorps de l'une quelconque des revendications 1 à 13.

**16.** Un ou plusieurs vecteurs contenant le ou les acides nucléiques selon la revendication 15.

**17.** Cellule hôte contenant le ou les acide(s) nucléique(s) selon la revendication 15 ou le ou les vecteur(s) selon la revendication 16.

**18.** Procédé de préparation d'un anticorps comprenant la culture de la cellule hôte selon la revendication 17 dans des conditions telles que le ou les acides nucléiques sont exprimés, et la récupération de l'anticorps à partir de la masse cellulaire ou du milieu de culture.

**19.** Anticorps selon l'une quelconque des revendications 1 à 13, pour utilisation dans le traitement d'un cancer, d'une maladie infectieuse, d'une maladie auto-immune, de l'asthme ou d'une maladie fibrotique.

# Figure 1

**Figure 2**

Figure 3

Figure 4

## Cal-51

Figure 5

**Figure 6**

## SKOV-3

## BT474

## SHP77

## Figure 7

Figure 8

## Figure 9

Figure 10

EP 3 049 440 B1

Figure 11

Figure 12A

Figure 12B

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011028952 A
- WO 2012025525 A
- WO 2009089004 A
- US 20070105199 A
- US 20090311253 A
- US 7083784 B
- US 20100234575
- US 7670600 B
- WO 2013096221 A
- US 7695936 B
- US 20030078385

- US 20100286374
- WO 2012125850 A
- US 4965195 A
- EP 0367566 A
- US 4968607 A
- EP 0460846 A
- US 7037784 B
- US 7371827 B
- US 2012070146 W
- US 20100183615
- US 20100150918

### Non-patent literature cited in the description

- **BARGOU et al.** *Science,* 2008, vol. 321 (5891), 974-977
- **HAAS et al.** *Immunobiology,* 2009, vol. 214 (6), 441-453
- Immunoglobulins: Structure and Function. **CARAYANNOPOULOS ; CAPRA.** Fundamental Immunology. Raven Press, 1993, 283-314
- **CARAYANNOPOULOS ; CAPRA.** FUNDAMENTAL IMMUNOLOGY. Raven Press, 1993, 284-286
- **MULDERMANS et al.** *J. Biotechnol.,* 2001, vol. 74, 277-302
- **DESMYTER et al.** *J. Biol. Chem.,* 2001, vol. 276, 26285-90
- **STRELTSOV et al.** *Protein Science,* 2005, vol. 14, 2901-2909
- BISPECIFIC ANTIBODIES. Springer, 2011
- **CANCER et al.** Principles and Practice of Oncology. J.B. Lippincott Co, 1993
- Hematology and Oncology, 144. Principles of Cancer Therapy. Merck Manual of Diagnosis and Therapy. 1999
- **CARAYANNOPOULOS ; CAPRA.** Immunoglobulins: Structure and Function. vol. 9, 283-314
- FUNDAMENTAL IMMUNOLOGY. Raven Press, 1993
- **EDELMAN et al.** *Proc. Natl. Acad. Sci.,* 1969, vol. 63, 78-85

- **KABAT et al.** SEQUENCES OF IMMUNOLOGICAL INTEREST. Public Health Service N.I.H, 1991
- **HONEGGER ; PLÜCKTHUN.** *J. Mol. Biol.,* 2001, vol. 309, 657-670
- **KABAT et al.** SEQUENCES OF PROTEINS OF IMMUNOLOGICAL INTEREST. Public Health Service N.I.H, 1991
- **NIELSEN et al.** *Protein Eng.,* 1997, vol. 10 (1), 1-6
- **VON HEIJNE.** *Eur. J. Biochem.,* 1983, vol. 133, 17-21
- **VON HEIJNE.** *J. Mol. Biol.,* 1985, vol. 184, 99-105
- **COSMAN et al.** *Nature,* 1984, vol. 312, 768
- **XU et al.** *Cell. Immunol.,* 2000, vol. 200 (1), 16-26
- **SCHELLER et al.** *Nature Biotechnol.,* 2001, vol. 19, 573-577
- **ZHU et al.** *Nature Biotechnol.,* 2005, vol. 23, 1159-1169
- **LAIBLE et al.** *Reprod. Fertil. Dev.,* 2012, vol. 25 (1), 315
- Cancer, Principles and Practice of Oncology. J. B. Lippincott Co, 1993
- **WARRENS et al.** *Gene,* 1997, vol. 186, 29-35
- **GUNASEKARAN et al.** *J. Biol. Chem.,* 2010, vol. 285, 19637-19646
- **WU et al.** *J. Biol. Chem.,* 2003, vol. 278, 25847-25852